(19) Europäisches Patentamt
European Patent Office
Office européen des brevets

(11) **EP 2 591 336 B1**

(12) **FASCICULE DE BREVET EUROPEEN**

(45) Date de publication et mention
de la délivrance du brevet:
**17.05.2017 Bulletin 2017/20**

(51) Int Cl.:
**G01N 21/45** (2006.01)     **G01N 21/78** (2006.01)
**G01N 21/77** (2006.01)     **C12Q 1/34** (2006.01)

(21) Numéro de dépôt: **11743307.8**

(22) Date de dépôt: **06.07.2011**

(86) Numéro de dépôt international:
**PCT/FR2011/051613**

(87) Numéro de publication internationale:
**WO 2012/004536 (12.01.2012 Gazette 2012/02)**

(54) **DISPOSITIF COLORIMETRIQUE POUR LA DETECTION, DANS UNE SOLUTION AQUEUSE D'INTERET, D'UNE ACTIVITE ENZYMATIQUE HYDROLYTIQUE A L'EGARD D'AU MOINS UN POLYMERE D'INTERET**

KOLORIMETRISCHE VORRICHTUNG FÜR DEN NACHWEIS VON HYDROLYTISCHER ENZYMATISCHER AKTIVITÄT IN EINER BESTIMMTEN WÄSSRIGEN LÖSUNG IM ZUSAMMENHANG MIT MINDESTENS EINEM BESTIMMTEN POLYMER

COLORMETRIC DEVICE FOR DETECTING, IN AN AQUEOUS SOLUTION OF INTEREST, HYDROLYTIC ENZYMATIC ACTIVITY WITH REGARD TO AT LEAST ONE POLYMER OF INTEREST

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.07.2010 FR 1055529**

(43) Date de publication de la demande:
**15.05.2013 Bulletin 2013/20**

(73) Titulaire: **Institut National de la Recherche Agronomique (INRA)
75007 Paris (FR)**

(72) Inventeurs:
• **CATHALA, Bernard**
  **F-44240 La Chapelle Sur Erdre (FR)**
• **CERCLIER, Carole**
  **F-44240 La Chapelle Sur Erdre (FR)**

(74) Mandataire: **Jacobacci Coralis Harle
14-16, rue Ballu
75009 Paris (FR)**

(56) Documents cités:
EP-A1- 0 677 738       US-A1- 2004 062 682
US-A1- 2007 297 944    US-A1- 2010 068 749

• YOKOTA ET AL: "Surface morphology of cellulose films prepared by spin coating on silicon oxide substrates pretreated with cationic polyelectrolyte", APPLIED SURFACE SCIENCE, ELSEVIER, AMSTERDAM, NL, vol. 253, no. 9, 7 février 2007 (2007-02-07), pages 4208-4214, XP005877309, ISSN: 0169-4332, DOI: DOI:10.1016/J.APSUSC.2006.09.037
• HABIBI ET AL: "Langmuir-Blodgett films of cellulose nanocrystals: Preparation and characterization", JOURNAL OF COLLOID AND INTERFACE SCIENCE, ACADEMIC PRESS, NEW YORK, NY, US, vol. 316, no. 2, 5 novembre 2007 (2007-11-05), pages 388-397, XP022328630, ISSN: 0021-9797, DOI: DOI:10.1016/J.JCIS.2007.08.041
• DUCERE V ET AL: "A capacitive humidity sensor using cross-linked cellulose acetate butyrate", SENSORS AND ACTUATORS B, ELSEVIER SEQUOIA S.A., LAUSANNE, CH, vol. 106, no. 1, 29 avril 2005 (2005-04-29), pages 331-334, XP025328551, ISSN: 0925-4005, DOI: DOI:10.1016/J.SNB.2004.08.028 [extrait le 2005-04-29]

**Description**

La présente invention concerne un dispositif pour la détection, dans une solution aqueuse d'intérêt, d'une activité enzymatique hydrolytique à l'égard d'au moins un polymère d'intérêt, avantageusement du type biopolymère.

## ETAT DE LA TECHNIQUE

Dans de nombreux domaines techniques et industriels, l'identification et l'étude d'enzymes ayant une activité hydrolytique envers des polymères constituent un enjeu majeur.

Par exemple, de telles enzymes hydrolytiques constituent un outil essentiel pour générer des biocarburants à partir de la biomasse riche en éléments lignocellulosiques.

Plus précisément, la paroi végétale est une structure complexe formée de polymères enchevêtrés (cellulose, hémicellulose, lignine, pectine, etc.) ; les enzymes hydrolytiques sont avantageusement employées pour rompre les chaines cellulosiques en des sucres destinés à être fermentés afin de créer du bioéthanol.

A cet effet, il est intéressant d'employer des dispositifs techniques qui permettent la détection, de manière rapide, simple et fiable, de l'activité hydrolytique d'une enzyme d'intérêt, ou d'une combinaison d'enzymes d'intérêt, envers un polymère ou une combinaison de polymères.

Il est en particulier courant de mettre en oeuvre des dispositifs colorimétriques dédiés, qui utilisent des réactions chimiques pour révéler l'activité enzymatique. Ces dispositifs colorimétriques chimiques emploient pour cela des colorants ou des indicateurs chimiques de détection.

Par exemple, une méthode chimique classiquement utilisée pour doser des activités de polysaccharides hydrolases est connue sous la dénomination de « méthode de Nelson » (décrite notamment dans Nelson et al., J. Biol. Chem. 1944, 153, 375-380).

Les dispositifs colorimétriques, pour la détection de l'activité hydrolytique envers un ou des polymères, sont ainsi souvent complexes à employer, nécessitent de nombreuses manipulations et réactifs, et obligent généralement l'emploi d'un dispositif de lecture (par exemple un spectroscope).

Pour détecter la présence d'un analyte dans un milieu d'intérêt, certains dispositifs colorimétriques utilisent un phénomène optique bien connu : l'interférence.

Ces dispositifs comportent pour cela au moins un film transparent mince ou une couche transparente mince, rapporté sur un substrat support.

L'épaisseur de ce film transparent mince est comprise dans une gamme de valeur permettant l'apparition d'une couleur particulière due au phénomène optique d'interférence, dite encore « couleur interférentielle » ou « couleur d'interférence ».

Ce phénomène s'explique par le fait qu'une partie du rayon incident est réfléchie sur l'interface air/film mince, tandis qu'une autre partie du rayon incident est réfractée puis réfléchie sur la deuxième interface film mince / substrat.

Les rayons réfléchis interagissent pour créer un phénomène optique d'interférence, consistant en des combinaisons entre des ondes lumineuses qui sont soit des interférences constructives, soit des interférences destructives.

La couleur résultante correspond à la longueur d'onde où le phénomène d'interférences constructives est maximal.

Un dispositif de ce type est par exemple décrit dans le document WO-A-2004/031760, prévu en particulier pour détecter des vapeurs organiques.

Le dispositif correspondant comprend pour cela trois couches superposées : - un substrat réflecteur, - une couche de détection apte à adsorber l'analyte et à changer d'épaisseur optique lors de l'exposition à l'analyte, et - une couche supérieure semi-réflective.

En pratique, l'analyte d'intérêt vient se fixer sur la couche de détection par adsorption, après avoir traversé le substrat réflecteur par porosité. Ce phénomène entraîne une modification de l'épaisseur de cette couche de détection, et par corollaire une modification de sa couleur interférentielle.

Un tel dispositif permet une détection d'un analyte, du fait de la fixation de ce dernier sur la couche de détection ; ce dispositif, et son procédé de mise en oeuvre, permettent uniquement une détection du genre analytique.

Le dispositif selon le document WO-A-2004/031760 ne vise aucunement à détecter les analytes en fonction de leurs activités biologiques.

De plus, ce dispositif de l'art antérieur n'est aucunement prévu, ni même adapté, pour la mise en oeuvre d'une détection fonctionnelle d'une activité enzymatique au sein d'une solution d'intérêt. En particulier, la couche supérieure semi-réflective est susceptible de constituer un obstacle à l'élimination de la couche de détection, en particulier par rinçage, qui serait dégradée par une enzyme.

Il existe par conséquent un besoin pour de tels dispositifs colorimétriques, employant un phénomène optique d'interférence sur couche mince de détection, qui soient aptes à détecter, dans une solution aqueuse d'intérêt, une activité enzymatique hydrolytique à l'égard d'au moins un polymère d'intérêt.

Le document US-A-2010/068749 décrit un tel dispositif colorimétrique, pour analyser l'âge et/ou la qualité de certains produits naturels, par exemple de denrées alimentaires.

Ce dispositif colorimétrique est constitué d'une superposition de couches, à savoir un support, un réflecteur, une couche polymère biodégradable et un miroir.

Ce dispositif colorimétrique est configuré de telle sorte qu'un changement de l'épaisseur de la couche polymère biodégradable aboutit à un changement de couleur visible à l'oeil nu, traduisant ainsi la présente d'une activité enzymatique.

A cet effet, la couche de polymère biodégradable est constituée d'un polymère qui est dégradable par des biomolécules, notamment des enzymes ou des métabolites catalytiques ; le miroir consiste quant à lui en une couche de nanoparticules.

Cependant, ce dispositif colorimétrique n'est pas entièrement satisfaisant ; et cette superposition particulière de couches, en particulier la présence de la couche miroir à base de nanoparticules, est relativement complexe à fabriquer.

## RESUME DE L'INVENTION

Dans ce contexte, la demanderesse a développé un dispositif colorimétrique permettant la détection, dans une solution aqueuse d'intérêt, d'une activité enzymatique hydrolytique à l'égard d'au moins un polymère d'intérêt, de manière simple, rapide et particulièrement sensible.

A cet effet, le dispositif selon l'invention est basé sur l'utilisation d'une couche transparente mince (ou film transparent mince), constituant un filtre d'interférence, pour la détection d'une activité enzymatique.

Cette couche de détection est apte à produire une couleur interférentielle qui est modifiée, qui apparaît ou qui disparaît, selon le cas, lors de l'exposition à une solution aqueuse comportant l'activité enzymatique hydrolytique recherchée.

A cet effet, le dispositif colorimétrique selon l'invention est caractérisé en ce qu'il comprend (i) un substrat délimité au moins par une surface supérieure, et (ii) une couche transparente de détection, ayant une première épaisseur $e$ et comprenant ledit polymère d'intérêt.

La couche transparente de détection comporte, d'une part, une surface supérieure, sur laquelle est destinée à être appliquée ladite solution aqueuse d'intérêt et formant une première interface air / couche de détection, et d'autre part, une surface inférieure, formée sur ladite surface supérieure du substrat et formant ensemble une seconde interface couche de détection / substrat. Lesdites deux interfaces sont aptes à générer un phénomène optique de réflexion.

La couche de détection est adaptée pour, d'une part, après l'application de ladite solution aqueuse d'intérêt lorsque celle-ci est dépourvue de ladite activité enzymatique hydrolytique, conserver ladite première épaisseur $e$, et d'autre part, suite à l'application de ladite solution aqueuse d'intérêt lorsque celle-ci comporte ladite activité enzymatique hydrolytique, présenter une seconde épaisseur $e'$, inférieure à ladite première épaisseur $e$,

Et ladite première épaisseur $e$ et/ou ladite seconde épaisseur $e'$ de ladite couche transparente de détection sont adaptées pour engendrer une couleur par un phénomène optique d'interférence généré par la recombinaison des faisceaux lumineux réfléchis auxdites interfaces air/couche de détection et couche de détection/substrat.

Selon une forme de réalisation préférée, la première épaisseur $e$ de la couche de détection est choisie pour engendrer une première couleur par un phénomène optique d'interférence ; et la couche de détection est adaptée pour, d'une part, après l'application de ladite solution aqueuse d'intérêt lorsque celle-ci est dépourvue de ladite activité enzymatique hydrolytique, conserver ladite première épaisseur $e$ engendrant ladite première couleur, et d'autre part, suite à l'application de ladite solution aqueuse d'intérêt lorsque celle-ci comporte ladite activité enzymatique hydrolytique, présenter une seconde épaisseur $e'$, inférieure à ladite première épaisseur $e$, engendrant soit une seconde couleur par un phénomène optique d'interférence, différente de ladite première couleur, soit la disparition de ladite première couleur.

D'autres caractéristiques avantageuses de l'invention, pouvant être prises en combinaison ou indépendamment les unes des autres, sont précisées ci-dessous :

- la première épaisseur $e$ de la couche transparente de détection est une épaisseur uniforme, choisie dans un domaine d'épaisseur allant de 70 à 900 nm, et de préférence entre 75 et 250 nm ;
- la première épaisseur $e$ et/ou la seconde épaisseur $e'$ de ladite couche transparente de détection sont adaptées pour engendrer une couleur par un phénomène optique d'interférence, avec une réflectance maximale pour une longueur d'onde comprise entre 380 nm et 780 nm, et de préférence entre 400 nm et 700 nm ;
- la couche transparente de détection a un indice de réfraction $n_c$ compris entre 1,2 et 1,7, et de préférence compris entre 1,4 et 1,6 ;
- le polymère contenu dans la couche supérieure de détection est choisi parmi les biopolymères, c'est-à-dire avantageusement les oligosaccharides, les polysaccharides, les peptides, les protéines, les lignines, les acides nucléiques, la cutine et/ou la subérine.

Selon un mode de réalisation particulier, le substrat est transparent, et la couche transparente de détection a un indice de réfraction $n_c$ qui est différent de l'indice de réfraction $n_s$ du substrat. Dans ce cas, l'indice de réfraction $n_s$ du substrat est avantageusement supérieur à l'indice de réfraction $n_c$ de la couche transparente de détection.

Selon un mode de réalisation alternatif, le substrat est opaque et comporte une surface supérieure réfléchissante.

Selon encore une caractéristique de réalisation, le polymère contenu dans la couche transparente de détection est avantageusement immobilisé, pour résister à l'application de la solution d'intérêt dépourvue d'activité enzymatique.

Dans ce cas, la couche supérieure de détection contient avantageusement des nanocristaux formant des réseaux de liaisons hydrogènes (« hornification ») avec le polymère d'intérêt.

De préférence, les nanocristaux consistent avantageusement en des nanocristaux de polysaccharides, et de préférence en des nanocristaux de cellulose.

Les nanocristaux correspondant ont avantageusement une charge de surface négative. Et la couche transparente de détection est constituée d'au moins deux premières sous-couches contenant chacune le polymère d'intérêt ; lesdites premières sous-couches sont séparées deux à deux par une sous-couche intercalaire contenant un composé poly-cationique.

Selon une forme de réalisation particulière, la couche transparente de détection est constituée d'au moins une paire de deux sous-couches (de préférence au moins deux paires de deux sous-couches superposées) contenant chacune au moins un polymère d'intérêt, avec une première sous-couche comportant un premier ou des premiers polymères d'intérêt (avantageusement les nanocristaux de polysaccharides), et une seconde sous-couches contenant un second ou des seconds polymères d'intérêt.

Toujours dans ce cas et de manière alternative, la couche transparente de détection contient avantageusement une résine formant des réseaux de liaisons covalentes avec le polymère d'intérêt.

La présente invention porte encore sur un procédé pour la détection, dans une solution aqueuse d'intérêt, d'une activité enzymatique hydrolytique à l'égard d'au moins un polymère d'intérêt.

Ce procédé est caractérisé en ce qu'il comprend au moins la succession d'étapes suivantes :

- la fourniture d'un dispositif colorimétrique tel que défini ci-dessus,
- l'application de ladite solution aqueuse d'intérêt sur la surface supérieure de la couche transparente de détection,
- le lavage de ladite surface supérieure de la couche transparente de détection, pour éliminer la solution aqueuse d'intérêt,
- le séchage de ladite couche transparente de détection, et
- l'analyse de la couleur de ladite couche transparente de détection au niveau du site d'application de ladite solution d'intérêt.

La présente invention porte encore sur l'utilisation d'un dispositif colorimétrique tel que défini ci-dessus, pour la détection, dans une solution aqueuse d'intérêt, d'une activité enzymatique hydrolytique à l'égard d'au moins un polymère d'intérêt.

## DESCRIPTION DES FIGURES

La présente invention est encore illustrée, sans être aucunement limitée par la description suivante en relation avec les figures annexées.

La figure 1 représente, de manière schématique et vue de côté, un dispositif colorimétrique selon l'invention, avant application de la solution aqueuse d'intérêt.

La figure 2 représente le dispositif colorimétrique selon la figure 1, après application d'une solution aqueuse d'intérêt comportant une activité enzymatique hydrolytique.

La figure 3 représente, de manière schématique et vue de côté, un dispositif colorimétrique comportant une couche mince de détection composée de plusieurs sous-couches, avant application de la solution aqueuse d'intérêt.

La figure 4 représente le dispositif colorimétrique selon la figure 3, après application d'une solution aqueuse d'intérêt comportant une activité enzymatique hydrolytique.

La figure 5 représente les courbes d'interférence, avant (trait discontinu) et après (trait continu) dégradation, pour une couche de détection à base de cellulose et de xyloglucane ; ces courbes correspondent à la réflectance, fonction de la longueur d'onde en nm.

## DESCRIPTION DETAILLEE DE L'INVENTION

La présente invention concerne ainsi un dispositif colorimétrique pour la détection, dans une solution aqueuse d'intérêt, d'une activité enzymatique hydrolytique à l'égard d'au moins un polymère d'intérêt.

Par « détection », on entend la discrimination entre la présence et l'absence, de l'activité enzymatique hydrolytique dans la solution aqueuse d'intérêt.

De manière alternative ou complémentaire, la « détection » de l'activité enzymatique hydrolytique consiste à déterminer une valeur quantitative d'activité enzymatique dans la solution aqueuse d'intérêt.

Cette « valeur quantitative » peut être exprimée en katal (kat), c'est-à-dire la quantité d'enzyme qui catalyse la transformation de 1 mole de substrat par seconde.

Cette « valeur quantitative » peut également être exprimée :

- en « unité internationale » (IU, International Unit), correspondant à la quantité d'enzyme qui catalyse la transformation de 1 μmole de substrat par minute,
- en « nombre de rotations » (kcat), qui est le nombre de molécules de substrat transformées par molécule d'enzyme par seconde (ou le nombre de moles de substrat transformé par mole d'enzyme),
- en « activité molaire » qui est le nombre de moles de substrat transformé par mole d'enzyme par minute,
- en « activité spécifique », soit l'activité par mg de protéine d'enzyme, exprimé en IU/mg protéine d'enzyme (ou μkat/mg).

Par « détection », on entend encore la discrimination entre la présence et l'absence, d'une enzyme active comportant ladite activité enzymatique hydrolytique, dans la solution aqueuse d'intérêt.

Par « activité enzymatique hydrolytique », on entend un mécanisme mis en oeuvre par une enzyme de type hydrolase, catalysant une réaction biochimique d'hydrolyse.

Des enzymes aptes à mettre en oeuvre de telles réactions d'hydrolyse sont en particulier classées dans le groupe EC 3 de la nomenclature dite « EC » (pour « Enzyme Commission numbers » - « Nomenclature Committee of the International Union of Biochemistry and Molecular Biology (NC-IUBMB) »).

Parmi les enzymes de type hydrolase, on peut en particulier citer les enzymes ayant une activité hydrolytique envers les biopolymères.

Parmi ces enzymes aptes à hydrolyser des biopolymères, on peut citer en particulier les polysaccharides hydrolases, dénommées encore glycoside hydrolases ou glycosidases (ces enzymes sont avantageusement classifiées dans le groupe EC 3.2.1).

Par « polysaccharides hydrolases », on entend par exemple les xylanases, les cellulases, les chitinases, les pectinases, les mannases, et la Cellulyve (marque déposée).

Parmi les enzymes hydrolytiques de biopolymères, on peut encore citer par exemple les protéases, les nucléases, les lignininases.

La « solution aqueuse d'intérêt » consiste en une phase liquide contenant plusieurs espèces chimiques, à savoir au moins (i) un solvant constitué majoritairement par de l'eau, de préférence entièrement par de l'eau, et (ii) un ou plusieurs composés en solution ou en suspension, appelés « soluté(s) ».

L'un au moins desdits composés en solution ou en suspension consiste en une enzyme active apte à générer ladite activité enzymatique hydrolytique, pour les solutions aqueuses comportant ladite activité.

Des exemples de polymères, envers lesquels l'activité enzymatique est recherchée, sont présentés ci-après.

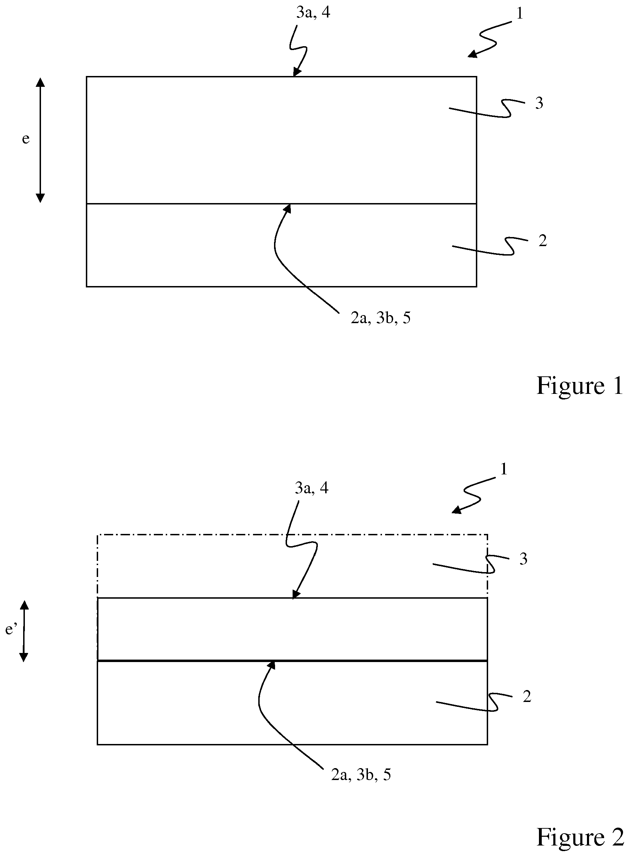

Le dispositif colorimétrique 1 selon l'invention est représenté schématiquement sur la figure 1.

Ce dispositif colorimétrique 1 comprend deux couches superposées, à savoir (i) un substrat 2, servant de support et délimité par une surface supérieure 2a, et (ii) une couche transparente de détection 3, comprenant le polymère d'intérêt et présentant un indice de réfraction désigné « $n_c$ ».

De manière générale, dans la présente description, les indices de réfraction donnés s'étendent pour une température de 20°C et pour une longueur d'onde de 520 nm.

La couche transparente de détection 3 est délimitée par deux surfaces opposées : (i) une surface supérieure 3a, sur laquelle est destinée à être appliquée ladite ou lesdites solutions aqueuses d'intérêts, et (ii) une surface inférieure 3b, formée sur ladite surface supérieure 2a du substrat 2.

La surface supérieure 3a de la couche transparente de détection 3 est libre, en ce sens qu'elle n'est aucunement recouverte par une couche additionnelle (par exemple une couche de nanoparticules).

La distance entre ces deux surfaces 3a et 3b de la couche de détection 3 définit l'épaisseur de ladite couche transparente de détection 3. Cette couche transparente de détection 3 comporte initialement une première épaisseur, désignée « e » (figure 1).

Ces couches 2 et 3 forment ensemble deux interfaces optiques, au niveau respectivement de la surface supérieure 3a et de la surface inférieure 3b de la couche de détection 3, à savoir :

- une première interface 4, située entre l'air et la couche de détection 3 (constituant un dioptre), et
- une seconde interface 5, entre la couche de détection 3 et le substrat 2.

Selon l'invention, la couche transparente de détection 3 est adaptée pour permettre la détection d'une activité enzymatique hydrolytique, envers le ou les polymères d'intérêt, dans une solution aqueuse étudiée. Cette couche de détection 3 constitue un moyen pour la traduction de ladite activité enzymatique hydrolytique détectée, en un signal optique.

A cet effet, la couche transparente de détection 3 est adaptée pour, après l'application de ladite solution aqueuse d'intérêt lorsque celle-ci est dépourvue de ladite activité enzymatique hydrolytique, conserver ladite première épaisseur e (figure 1). Cette même couche transparente de détection 3 est également adaptée pour, suite à l'application de la solution aqueuse d'intérêt lorsque celle-ci comporte ladite activité enzymatique hydrolytique, présenter une seconde épaisseur e', inférieure à ladite première épaisseur e (figure 2).

La présence d'une activité enzymatique hydrolytique dans la solution aqueuse d'intérêt est ainsi apte à provoquer une réduction de l'épaisseur de la couche de détection 3 (depuis la première épaisseur e jusqu'à la seconde épaisseur e'), qui aboutit à un changement de la coloration observable (directement par un opérateur ou au moyen d'un dispositif approprié) au niveau de cette couche transparente de détection 3.

Pour cela, la première épaisseur e de la couche transparente de détection 3 et/ou la seconde épaisseur e' de la couche transparente de détection 3 sont aptes à engendrer une couleur par un phénomène optique d'interférence.

Tel que développé par la suite, ces caractéristiques du dispositif 1 permettent l'observation d'un changement optique au niveau de la couche de détection 3 en présence d'une activité enzymatique d'intérêt.

Ce changement optique consiste avantageusement en l'un des phénomènes suivants :

(i) le passage d'une première couleur interférentielle à une seconde couleur interférentielle, ou
(ii) l'apparition d'une couleur interférentielle, ou (iii) la disparition d'une couleur interférentielle.

En effet, la couche transparente de détection 3 intervient en tant que filtre d'interférence. En fonction de son épaisseur e ou e', la couche transparente de détection 3 comporte une couleur due à des phénomènes optiques de réflexions et d'interférences (constructives/destructives) pour des longueurs d'ondes particulières, avantageusement dans le domaine visible.

Plus précisément, lorsque la lumière rencontre la première interface 4 (air/couche de détection 3), une partie du faisceau est réfléchie. L'autre partie de la lumière est transmise à travers la couche de détection 3, pour être réfléchie sur la deuxième interface 5 (couche de détection 3/substrat 2).

Le rayon réfléchi sur la deuxième interface 5 parcourt une distance plus grande, provoquant un déphasage de la lumière. Lorsque la distance parcourue est de l'ordre de grandeur de la moitié de la longueur d'onde de la lumière incidente, la recombinaison des faisceaux réfléchis provoque une interférence qui conduit à l'apparition d'une couleur au niveau de la couche de détection 3.

On parlera ainsi indifféremment de la « couleur d'interférence » ou de la « couleur interférentielle », que l'on peut observer au niveau de la couche de détection 3.

Par « couleur » de la couche de détection 3, en particulier d'une couleur interférentielle, on entend une couleur perçue (avantageusement directement par un individu, à « l'oeil nu ») possédant (i) une tonalité chromatique (la tonalité chromatique caractérise la couleur en elle-même, c'est-à-dire rouge, vert, jaune, bleu, etc.) et/ou (ii) une clarté et/ou (iii) une saturation.

Par « couleur », en particulier une couleur interférentielle, on entend encore la longueur d'onde où le phénomène d'interférences constructives est maximal ; cette couleur correspond éventuellement encore à la longueur d'onde où la courbe de réflectance est maximale, avantageusement dans le domaine du visible ; cette couleur peut également être définie par sa courbe spectrale (réflectance en fonction de la longueur d'onde).

L'interférence, et par conséquent l'apparition de la couleur, est donc liée en substance (i) à l'épaisseur de la couche de détection 3 et (ii) aux phénomènes de réflexion aux deux interfaces 4, 5 délimitant ladite couche de détection 3. Cette couleur ne nécessite aucun marquage chimique du polymère.

En pratique et comme évoqué ci-dessus, en fonction de la première épaisseur e et de la seconde épaisseur e' de la couche de détection 3, ce changement de coloration consiste avantageusement en une apparition, en une disparition ou en une modification de la couleur interférentielle au niveau de la couche de détection 3.

Ce « changement de coloration » consiste alors avantageusement en un changement de (i) la tonalité chromatique et/ou (ii) la clarté et/ou (iii) la saturation, observable directement par un individu.

Pour être complet, différents aspects du dispositif colorimétrique 1 sont présentés plus en détail ci-dessous.

SUBSTRAT

Le substrat 2 est réalisé dans un matériau apte (i) à constituer un support pour la couche transparente de détection, et (ii) à assurer une réflexion (totale ou partielle, et avantageusement spéculaire) de faisceaux lumineux appliqués au dispositif colorimétrique 1.

Selon un premier mode de réalisation, le substrat 2 consiste en un matériau transparent, comportant un indice de réfraction désigné « $n_s$ ». Ce matériau est avantageusement du type milieu linéaire homogène isotrope.

L'indice de réfraction $n_s$ de ce substrat 2 est différent de l'indice de réfraction $n_c$ de la couche transparente de détection 3. La seconde interface 5, entre la couche de détection 3 et le substrat 2, constitue ainsi un dioptre.

En particulier, le matériau constitutif du substrat 2 est choisi avec un indice de réfraction $n_s$ supérieur à l'indice de réfraction $n_c$ de la couche transparente de détection 3.

Par exemple, pour une couche supérieure de détection 3 comportant un indice de réfraction $n_c$ compris entre 1,2 et 1,7, le substrat 2 consiste avantageusement en une tranche de silicium (couramment dénommée « wafer ») avec un indice de réfraction compris entre 3 et 5.

De manière générale, plus la différence entre les indices de réfraction $n_c/n_s$ de cette interface 5 (entre la couche de détection 3 et le substrat 2) est élevée, plus le coefficient de réflexion (pourcentage de lumière réfléchie) est élevé.

Selon un second mode de réalisation, le substrat 2 consiste en un matériau opaque comportant une surface supérieure 2a présentant des propriétés réfléchissantes.

Par « réfléchissante », on entend ici une réflexion optique totale ou au moins semi-réfléchissante.

Des matériaux adaptés pour obtenir une telle surface supérieure réfléchissante 2a incluent les éléments chimiques métalliques tels que l'aluminium, le chrome, l'or, le nickel, le silicium, l'argent ou leur mélange. D'autres matériaux adaptés incluent les oxydes métalliques, tels que l'oxyde de chrome et l'oxyde de titane.

De manière générale et quel que soit le mode de réalisation, le coefficient de réflexion au niveau de la seconde interface 5 est par exemple d'au moins 20 %, éventuellement compris entre 20% et 90%, et éventuellement supérieur à 90%.

## COUCHE TRANSPARENTE DE DETECTION

La couche de détection 3 comprend au moins un polymère d'intérêt, c'est-à-dire un unique polymère ou une combinaison de polymères.

Par « polymère », on englobe aussi bien les copolymères que les homopolymères.

Le polymère est avantageusement choisi parmi les biopolymères, ou autrement dit les « polymères organiques », c'est-à-dire les polymères issus d'un édifice biologique.

A titre indicatif, ces polymères peuvent être choisis parmi :

- les oligosaccharides et polysaccharides (cellulose, xylane, pectine, chitosan, chitine, xyloglucane, arabinoxylane, etc.),
- peptides et protéines (sérum albumine bovine ou humaine, gluténine, etc.),
- les acides nucléiques, notamment les acides désoxyribonucléiques, les acides ribonucléiques, etc.),
- la cutine, la subérine et la lignine.

De tels biopolymères peuvent se présenter sous une forme isolée (c'est-à-dire des chaînes de polymères distinctes), agrégée (c'est-à-dire des chaînes de polymères entremêlées) ou cristallisée (c'est-à-dire des chaînes de polymères organisées selon un motif répétitif ordonné).

La couche de détection 3 comporte un indice de réfraction $n_c$ qui est avantageusement compris entre 1,2 et 1,7, et de préférence compris encore entre 1,4 et 1,6.

La surface supérieure 3a et la surface inférieure 3b de la couche de détection 3 sont parallèles, ou au moins approximativement parallèles, l'une par rapport à l'autre.

Ces deux surfaces 3a et 3b sont de préférence planes, ou au moins approximativement planes.

Comme évoqué ci-dessus, ces deux surfaces 3a et 3b définissent la première épaisseur e, qui consiste avantageusement en une épaisseur uniforme ou constante sur toute la surface.

Cette première épaisseur e est choisie avantageusement dans un domaine compris entre 70 et 900 nm, et de préférence encore compris entre 75 et 250 nm.

Comme indiqué précédemment, la couche transparente de détection 3 est apte à conserver ladite première épaisseur e lors de l'application d'une solution dépourvue de ladite activité enzymatique.

A cet effet, le ou les polymères d'intérêt sont avantageusement immobilisés.

Cette immobilisation peut être générée par des liaisons (i) de type covalentes ou « chimiques » (réticulation) et/ou (ii) de type non covalentes ou « physiques » (électrostatiques, hydrogènes, force de Van der Waals).

Dans le cas (i) ci-dessus, la couche supérieure de détection 3 contient avantageusement une résine formant des réseaux de liaison covalente avec le ou les polymères d'intérêt.

Cette résine est choisie en fonction du ou des polymères à réticuler.

Pour le choix et la mise en oeuvre d'une telle résine, on peut se référer par exemple au document suivant : Ducéré et al., 2005 (« A capacitive humidity sensor using cross-linked cellulose acetate butyrate » Sensors and Actuators B: Chemical 106 (1), 331-334 (2005)), Wu et al., 2009 (« Molecularly imprinted organic-inorganic hybrid membranes for selective séparation of phenylalanine isomers and its analogue » Séparation and Purification Technology 68 (1), 97-104 (2009)), et Schuler et al., 2001 (« Decomposable hollow biopolymer-based capsules » Biomacromolecules 2 (3), 921-926 (2001)).

Par exemple, la réticulation par des résines mélamine-urée-formaldéhyde (désignées encore « MUF ») présente les avantages suivants :

- la réaction de réticulation peut avoir lieu avec de nombreuses fonctions chimiques (alcool, amine, phénol), permettant ainsi de réticuler un grand nombre de classes de biopolymères ;
- il existe des formulations de résine soluble dans l'eau, permettant une mise en oeuvre simple par un mélange de la solution devant être déposée ;
- la réticulation est simple : le monomère est stable à température ambiante et il réagit lorsque le film est porté à 90°C en atmosphère sèche pendant une heure, ce traitement étant compatible avec la majorité des biopolymères.

Dans le cas (ii) ci-dessus, l'immobilisation peut être obtenue au moyens de nanocristaux formant des réseaux de liaisons hydrogènes (encore connus sous l'appellation du phénomène de « hornification ») avec le polymère d'intérêt.

Pour cela, les nanocristaux consistent avantageusement en des composés comportant des charges électrostatiques négatives en surface. On peut pour cela choisir de préférence des nanocristaux de polysaccharides, et de préférence des nanocristaux de cellulose.

De tels nanocristaux sont par exemple préparés par hydrolyse acide du coton selon le protocole décrit dans Revol et al. (Int. J. Biol. Macromol. 1992, 14, 170-172.) ou Cranston et al. (Biomacromolecules, vol 7, 2006, p2522).

Par exemple, pour stabiliser une couche transparente de détection 3 à base de xyloglucane, on ajoute des nanocristaux de cellulose. Une couche transparente de détection 3 à base d'arabinoxylane est quant à elle fixée avantageusement à l'aide d'une résine mélamine-urée-formaldéhyde.

D'autre part, la couche de détection 3 peut consister en une couche unique formant un milieu linéaire homogène isotrope (ou « MLHI »). Dans ce cas, la couche de détection 3 est avantageusement constituée par le ou les polymères d'intérêt sur toute sa hauteur.

De manière alternative, la couche transparente de détection 3 peut être constituée de plusieurs sous-couches superposées, l'une au moins desdites sous-couches contenant le ou les polymères d'intérêt.

Dans ce dernier cas, la sous-couche contenant le ou les polymères d'intérêt constitue avantageusement au moins la surface supérieure 3a de la couche de détection 3.

La couches de détection 3 comportent alors avantageusement au moins deux sous-couches, encore avantageusement entre huit et seize sous-couches.

Dans ce cadre, la couche de détection 3 est constituée avantageusement de plusieurs paires de sous-couches superposées, avantageusement entre quatre et huit paires de sous-couches superposées. Lesdites paires de sous-couches superposées sont identiques, ou au moins approximativement identiques, entre elles.

Dans le cas d'une telle structure à plusieurs sous-couches, la couche de détection 3 peut comporter une alternance de polymères d'intérêt, formant les paires de sous-couches superposées, avec par exemple successivement une sous-couche de cellulose (avantageusement sous forme de nanocristaux) et une sous-couche de xyloglucane.

Selon un autre exemple, la couche de détection 3 peut comporter une alternance de sous-couches, formant les paires de sous-couches superposées, avec successivement une sous-couche de polyélectrolyte (par exemple poly-L-lysine ou « PLL », ou chlorhydrate de poly(allylamine) ou « PAH ») et une sous-couche comprenant le ou les polymères d'intérêt (par exemple un mélange de cellulose et de xyloglucane).

L'alternance de sous-couches permet en particulier d'atteindre l'épaisseur e voulue au niveau de la couche de détection 3, comme développé ci-après dans le cadre du procédé de dépose.

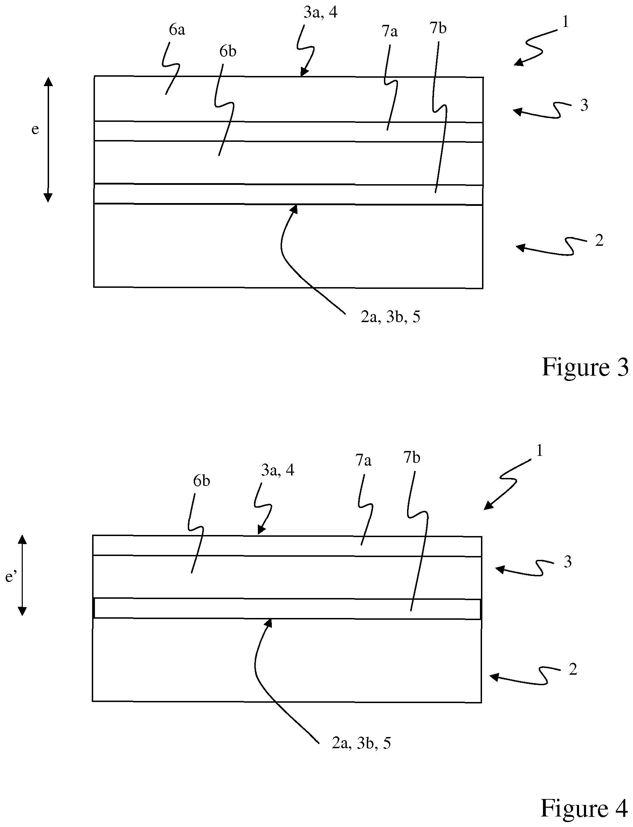

Par ailleurs, dans le cas d'un substrat 2 et d'une couche de détection 3 comportant une même charge électrostatique, la face supérieure 2a du substrat 2 est avantageusement revêtue d'une sous-couche d'accroche constituée d'un polyélectrolyte (représentée par exemple sur la figure 3 et désignée par le repère 7b), pour créer une interface de liaison entre ledit substrat 2 et ladite couche de détection 3.

Par exemple, la sous-couche de polyélectrolyte consiste en de la poly-L-lysine ou du chlorhydrate de poly(allylamine). C'est avantageusement le cas, d'une part, pour un substrat constitué par une tranche de silicium et, d'autre part, pour une couche de détection ou une sous-couche contenant des nanocristaux de cellulose, qui sont tous deux chargés négativement.

## PROCEDE DE FABRICATION

La couche transparente de détection 3 est déposée sur le substrat 2 par une technique appropriée, en une ou plusieurs étapes.

De manière générale, il existe différentes techniques pour construire la couche transparente de détection 3.

La technique de trempage (ou « dip-coating ») consiste à tremper le substrat dans une solution de polymères. Cette méthode permet d'obtenir des couches très fines, de quelques nanomètres.

Pour mettre en oeuvre cette technique, on peut se référer par exemple aux documents suivants : Jean et al., 2008 (« Structural Details of Cellulose Nanocrystals/Polyelectrolytes Multilayers Probed by Neutron Reflectivity and AFM » Langmuir 24 (7), 3452-3458 (2008)), ou Cranston et al., 2006 (« Morphological and Optical Characterization of Polyelectrolyte Multilayers Incorporating Nanocrystalline Cellulose » Biomacromolecules 2006, 7, (9), 2522-2530).

La pulvérisation (ou « spray-coating ») consiste à pulvériser le polymère sur le support. Le solvant est évaporé en partie pendant la pulvérisation et le reste s'évapore après avoir été déposé. Cette technique a l'intérêt d'être plus rapide que le « dip-coating ».

Cette technique est par exemple décrite dans le document Wagberg et al. « The Build-Up of Polyelectrolyte Multilayers of Microfibrillated Cellulose and Cationic Polyelectrolytes » Langmuir 24 (3), 784-795 (2008).

L'enduction centrifuge (dite encore tournette ou « spin-coating ») consiste à déposer la solution contenant le polymère sur le substrat, de préférence au centre de ce dernier. Le substrat en rotation provoque l'étalement de la solution déposée sur sa surface et l'évacuation de l'excédent de liquide, par centrifugation ; le solvant résiduel s'évapore, ne laissant que la matière sur le substrat. L'épaisseur de la couche mince obtenue varie avec la vitesse de rotation, la viscosité et/ou la concentration de la solution.

Cette dernière technique est par exemple étudiée dans les documents Cranston et al., « Morphological and Optical Characterization of Polyelectrolyte Multilayers Incorporating Nanocrystalline Cellulose » Biomacromolecules 2006, 7, (9), 2522-2530, ou Cranston et al, « Birefringence in Spin-Coated Films Containing Cellulose Nanocrystals » Colloids and Surfaces A 2008, 325, (1-2), 44-51.

Dans le cadre de cette dernière technique, pour ajuster l'épaisseur de la couche ou des sous-couches destinées à constituer la couche de détection 3, il est avantageusement possible de se référer par exemple à la formule (1) suivante, issue de David et a., « Spin Coating of Thin and Ultrathin Polymer Films » Polymer engineering and science, vol. 38, N° 12, December 1998, p 2040, à savoir:

$$(1) \qquad h_f \propto \left( \frac{\eta_0}{\rho\omega} \right)^{1/2}$$

avec $h_f$ : épaisseur de la couche
$\eta_o$ : viscosité initiale de la solution
$\rho$ : densité du liquide
$\omega$ : vitesse de rotation

Ce principe est valable en particulier pour les polyélectrolytes, mais s'applique aussi pour des matériaux ayant d'autres types d'interactions entre eux.

En pratique, la couche de détection 3 est avantageusement déposée en plusieurs étapes, pour constituer une superposition de sous-couches. Une telle procédure est par exemple développée dans la partie Exemple ci-après.

Cette démarche permet d'optimiser la fabrication et la stabilité de la couche de détection 3. Une telle démarche peut également être utile pour introduire des sous-couches dans son épaisseur, qui visent à définir l'épaisseur finale e' de la couche de détection 3, après action enzymatique.

PROCEDE POUR LA DETECTION D'UNE ACTIVITE ENZYMATIQUE

En pratique, le procédé pour la détection, dans une solution aqueuse d'intérêt, d'une activité enzymatique hydrolytique à l'égard d'au moins un polymère d'intérêt, comprend au moins la succession d'étapes suivantes.

Après la fourniture d'un dispositif colorimétrique 1 tel que défini ci-dessus en relation avec la figure 1, la solution aqueuse d'intérêt est appliquée sur la surface supérieure 3a de la couche transparente de détection 3.

Cette solution aqueuse est déposée sous la forme d'une goutte, dont le volume est avantageusement compris entre $20\mu L$ et 1 mL.

Le dispositif colorimétrique 1 est alors incubé pendant un temps et des conditions appropriés, en fonction notamment de l'activité enzymatique étudiée.

La surface supérieure 3a de la couche transparente de détection 3 est ensuite lavée, pour éliminer la solution aqueuse d'intérêt et pour éliminer la partie de la couche de détection ayant éventuellement subie une hydrolyse enzymatique.

On utilise pour cela avantageusement une solution aqueuse dépourvu d'activité enzymatique, par exemple de l'eau ou un tampon de dissolution approprié.

On procède ensuite au séchage de ladite couche transparente de détection 3.

Pour finir, la couleur de la couche transparente de détection 3, au niveau du site d'application de ladite solution d'intérêt, est analysée.

A cet effet, une source lumineuse (ou un faisceau lumineux) est orientée en direction de la couche transparente de détection 3.

Cette source de lumière peut être naturelle ou artificielle. Cette lumière est avantageusement du type polychromatique, et de préférence encore du type lumière à spectre continu.

Sauf stipulation contraire, le dispositif colorimétrique 1 est observé selon un angle normal à la surface supérieure 2a du substrat 2. D'autres angles de vues pourraient être utilisés, par exemple inférieurs à 30°, et de préférence encore inférieurs à 15°, par rapport à un axe perpendiculaire à la surface supérieure 2a du substrat 2.

Comme évoqué précédemment, en l'absence d'une activité enzymatique hydrolytique dans la solution aqueuse d'intérêt, la couche transparente de détection 3 conserve l'épaisseur $e$ initiale. Il n'y a alors pas de changement optique au niveau de la couche de détection 3.

En revanche, la présence d'une activité enzymatique hydrolytique dans la solution aqueuse d'intérêt entraîne une dégradation de la couche transparente de détection 3, qui aboutit à une diminution de son épaisseur.

Dans ce cas, la couche transparente de détection 3, initialement d'une première épaisseur $e$ (figure 1), termine à une seconde épaisseur $e'$ (figure 2) qui est inférieure à ladite première épaisseur $e$.

Cette seconde épaisseur $e'$ est avantageusement comprise entre 0 (la couche de détection 3 est éliminée) et 250 nm, et de préférence entre 70 et 250 nm.

Ce changement d'épaisseur, depuis la première épaisseur $e$ jusqu'à la seconde épaisseur $e'$, provoque alors avantageusement l'un des changements optiques suivants :

(i) le passage depuis une première couleur interférentielle jusqu'à une seconde couleur interférentielle, lesdites couleurs interférentielles étant différentes l'une de l'autre,
(ii) le passage depuis une absence de couleur interférentielle jusqu'à l'apparition d'une couleur interférentielle, ou
(iii) le passage depuis une couleur interférentielle jusqu'à la disparition de ladite couleur interférentielle.

La lecture de la couleur de la couche de détection 3 peut être réalisée (i) directement par l'opérateur ou (ii) au moyen d'un système de lecture adapté.

Le changement de la couleur de la couche transparente de détection 3 est avantageusement observé dans le domaine de la lumière visible, et peut ainsi être détecté directement par un individu, sans faire appel à un dispositif de lecture.

Dans ce cas, la première épaisseur $e$ et/ou la seconde épaisseur $e'$ de la couche de détection 3 sont choisies de sorte à obtenir une couleur interférentielle dont la réflectance maximale se situe à une longueur d'onde comprise entre 380 nm et 780 nm, et de préférence comprise entre 400 nm et 700 nm.

Plus généralement, le changement optique consiste avantageusement un changement de la tonalité chromatique, ce qui facilite la lecture et l'interprétation des résultats.

Pour faciliter cette lecture directement par un opérateur, on peut encore appliquer une solution aqueuse témoin sur la même couche de détection 3, à proximité du site d'application de la solution aqueuse d'intérêt. Cette solution aqueuse témoin consiste avantageusement en une solution aqueuse dépourvue de l'activité enzymatique (par exemple par inactivation de l'activité enzymatique, avantageusement à la chaleur).

Une couleur différente entre le site d'application de la solution d'intérêt et le site d'application de la solution aqueuse témoin montre la présence d'une activité enzymatique dans ladite solution d'intérêt. Une couleur identique entre le site d'application de la solution d'intérêt et le site « témoin » démontre l'absence d'une activité enzymatique dans la solution d'intérêt.

Si nécessaire, des dispositifs de lecture peuvent être utilisés pour observer un changement de la couleur de la couche transparente de détection, notamment lorsque le dispositif colorimétrique est soumis à d'autres sources lumières telles que les rayonnements ultraviolets (UV), les rayonnements infrarouges ou les rayonnements infrarouges proches.

A cet effet, des dispositifs de lecture peuvent être utilisés pour l'analyse des dispositifs colorimétriques 1, par exemple des photodétecteurs, y compris des dispositifs à transfert de charge (encore désignés Charge-coupled Device ou CCD), des caméras numériques, etc.

Dans ce cas, le dispositif colorimétrique 1 est employé dans un système colorimétrique automatisé comprenant avantageusement un dispositif de lecture optique, une source lumineuse, et optionnellement, des moyens pour analyser le changement de couleur.

L'activité enzymatique peut alors être recherchée par une comparaison des couleurs obtenues (i) par la solution d'intérêt et par une solution « témoin » ou (ii) avant et après application de la solution aqueuse d'intérêt.

Les dispositifs de lecture permettent alors avantageusement de déterminer le spectre de réflectance visible au niveau de la couche de détection 3. De manière avantageuse et à titre d'exemple, on peut considérer qu'il y a un changement optique lorsque la longueur d'onde de réflectance maximale au niveau de ladite couche de détection 3 est différente (i) entre la solution d'intérêt et la solution « témoin » ou (ii) avant et après application de la solution aqueuse d'intérêt.

Une couleur différente avant et après application de la solution aqueuse d'intérêt montre la présente d'une activité enzymatique dans ladite solution d'intérêt. Une couleur identique, ou approximativement identique, avant et après application de la solution aqueuse d'intérêt montre l'absence d'une activité enzymatique dans la solution d'intérêt.

De plus, pour déterminer une valeur quantitative d'activité enzymatique dans la solution aqueuse d'intérêt, il suffit d'adapter par exemple les dilutions de la solution d'intérêt et/ou le temps d'incubation et/ou la structure de la couche de détection. On peut alors envisager que la couleur finale soit fonction de ladite valeur quantitative.

MODES DE REALISATION PARTICULIERS

Selon un premier mode de réalisation correspondant à la figure 1, la couche transparente de détection 3 est avantageusement constituée par un milieu linéaire homogène isotrope (ou « MLHI »), déposé en une ou plusieurs étapes.

Dans ce cas, la diminution d'épaisseur de la couche de détection 3 (depuis l'épaisseur $e$ initiale sur la figure 1 jusqu'à l'épaisseur $e'$ finale sur la figure 2) dépend principalement du temps d'incubation.

De manière alternative et tel que représenté sur la figure 3, la couche transparente de détection 3 peut être constituée d'une superposition de quatre sous-couches 6 et 7 :

- deux des sous-couches 6$\underline{a}$ et 6$\underline{b}$ comportent chacune le ou les polymères d'intérêt, avantageusement immobilisés par des nanoparticules anioniques, et
- deux autres des sous-couches 7$\underline{a}$ et 7$\underline{b}$, contiennent chacune un composé polycationique.

Chaque sous-couche 6, 7 constitue un milieu linéaire homogène isotrope (ou « MLHI »).

Ces sous-couches 6 et 7 sont déposées alternativement, formant des paires de sous-couches 6$\underline{a}$, 7$\underline{a}$ et 6$\underline{b}$, 7$\underline{b}$ superposées, avec en particulier une sous-couche de polymère(s) 6 formant la surface supérieure 3$\underline{a}$ de la couche de détection 3.

Cette structure vise notamment à pallier aux difficultés pour obtenir des sous-couches individuelles épaisses ; l'alternance de sous-couches permet d'obtenir l'épaisseur $e$ initiale souhaitée.

Par exemple, les sous-couches polycationique 7 sont constituées de chlorhydrate de poly(allylamine) ou de poly-L-lysine.

Dans ce cas, la diminution d'épaisseur de la couche de détection 3 (depuis l'épaisseur $e$ initiale sur la figure 3 jusqu'à l'épaisseur $e'$ finale sur la figure 4) dépend avantageusement de la sous-couche de polymère(s) 6$\underline{a}$ constituant la surface supérieure 3$\underline{a}$ de la couche de détection 3.

En d'autres termes, l'activité enzymatique hydrolytique assure de préférence une dégradation totale de la sous-couche de polymère(s) 6$\underline{a}$ constituant initialement la surface supérieure 3$\underline{a}$ de la couche de détection 3 (tel que représenté sur la figure 4). Après cette action enzymatique, la surface supérieure 3$\underline{a}$ de la couche de détection 3 est constituée par la surface supérieure de la sous-couche polycationique 7$\underline{a}$ directement sous-jacente.

Ce mode de réalisation présente l'intérêt de permettre un contrôle de l'épaisseur $e$ initiale, et aussi de l'épaisseur $e'$ finale générée en présence d'une activité enzymatique.

Encore de manière alternative et conformément à la figure 3, la couche transparente de détection 3 peut être constituée d'une superposition de sous-couches 6 et 7 :

- deux des sous-couches 6$\underline{a}$ et 6$\underline{b}$ comportent chacune un premier ou des premiers polymères d'intérêt, et
- deux autres des sous-couches 7$\underline{a}$ et 7$\underline{b}$, contiennent chacune un second ou des seconds polymères d'intérêt.

Ces sous-couches 6 et 7 sont déposées alternativement, formant des paires de sous-couches 6$\underline{a}$, 7$\underline{a}$ et 6$\underline{b}$, 7$\underline{b}$ superposées.

Par exemple, le premier polymère d'intérêt consiste en des nanocristaux de cellulose ; et le second polymère d'intérêt consiste en du xyloglucane.

De manière générale, le dispositif colorimétrique selon l'invention est ainsi adaptable pour la détection d'activités hydrolytiques à l'égard d'un polymère d'intérêt.

Ce dispositif a pour avantage (i) de ne pas nécessiter de matériels complexes pour son utilisation, (ii) de présenter un fort potentiel de miniaturisation et (iii) de pouvoir être intégrer dans un système d'analyse à haut débit.

Il est également intéressant en ce qu'il peut être employé dans le cadre d'une mesure semi-quantitative d'une



EP 2 591 336 B1

activité hydrolytique, en adaptant notamment l'épaisseur de la couche de détection et le temps de réaction.

Par exemple, un procédé possible pour une mesure semi-quantitative d'une telle activité hydrolytique dans une solution aqueuse comprend avantageusement les étapes suivantes :

- la fourniture d'un dispositif colorimétrique selon l'invention, dont la couche transparente de détection est constituée par au moins un polymère d'intérêt apte à subit une hydrolyse en présence de ladite activité hydrolytique,
- l'application de ladite solution aqueuse d'intérêt sur la surface supérieure de la couche transparente de détection dudit dispositif colorimétrique,
- le lavage de ladite surface supérieure de la couche transparente de détection,
- le séchage de ladite couche transparente de détection, et
- l'analyse de la couleur de ladite couche transparente de détection au niveau dudit site d'application de ladite solution d'intérêt, en comparaison d'un ou plusieurs sites témoin (par exemple une gamme d'étalonnage), pour l'évaluation semi-quantitative d'activité hydrolytique dans ladite solution aqueuse.

Ce dispositif colorimétrique selon l'invention a aussi l'intérêt de pouvoir permettre l'identification d'une activité hydrolytique spécifique à l'égard d'un polymère déterminé.

Par exemple, un procédé envisageable pour identifier une telle activité hydrolytique spécifique dans une solution aqueuse comprend avantageusement les étapes suivantes :

- la fourniture d'un dispositif colorimétrique selon l'invention, dont la couche transparente de détection est constituée par un polymère d'intérêt apte à subit une hydrolyse en présence de ladite activité hydrolytique,
- l'application de ladite solution aqueuse d'intérêt sur la surface supérieure de la couche transparente de détection dudit dispositif colorimétrique,
- le lavage de ladite surface supérieure de la couche transparente de détection,
- le séchage de ladite couche transparente de détection, et
- l'analyse de la couleur de ladite couche transparente de détection au niveau dudit site d'application de ladite solution d'intérêt, et
- la comparaison de la couleur dudit site d'application avec un site témoin, pour déterminer la présence ou l'absence de ladite activité hydrolytique,
- la sélection positive de ladite solution aqueuse, ou de la ou des enzymes qu'elle contient, si la comparaison montre que ladite solution aqueuse testée modifie convenablement la couleur de ladite couche transparente de détection.

La présente invention est encore illustrée par les exemples développés ci-dessous.

## EXEMPLES

### Exemple 1 : Réalisation d'une couche transparente de détection sur un substrat, et étude de sa stabilité

Matériel

Les différents produits utilisés sont :

- l'arabinoxylane de blé haute viscosité (AX-HV), 47 cSt (commercialisé par Megazyme)
- l'arabinoxylane de blé moyenne viscosité (AX-MV), 22 cSt (commercialisé par Megazyme)
- le chlorhydrate de poly(allylamine) (PAH) (commercialisé par SIGMA)
- des nanocristaux de cellulose (C) (encore désignés sous l'appellation de « whiskers ») préparés par hydrolyse acide du coton selon le protocole décrit dans Cranston et al (Biomacromolecules, vol 7, 2006, p2522)
- le xyloglucane (XG) extrait de graine de Tamarin (Silvestre Virginie, thèse 2004, Université de Nantes),
- une résine mélamine formaldéhyde (MUF) Resimene AQ-7550 (commercialisé par INEOS Melamines) ; cette résine est préalablement diluée à 10% à partir de 640 $\mu$L de solution commerciale et complétée à 5 mL d'HCl à $10^{-2}$ mol/L,
- une tranche de silicium (encore couramment désignée sous l'appellation de « wafer ») comportant une surface supérieure polie.

Méthodes

1.1. Réalisation des couches minces

1.1.1.- Construction par enduction centrifuge

Le support est nettoyé dans un mélange corrosif composé de 70% d'acide sulfurique $H_2SO_4$ et 30% de peroxyde d'hydrogène $H_2O_2$ pendant 30 minutes.
Ensuite le support est rincé dans deux bains d'eau successifs et séché à l'azote.
Les couches ont été réalisés par le dispositif pour enduction centrifuge Spin 150 NPP (SPS Europe) à une vitesse de rotation de 2000 rpm (pour « rotations par minute ») avec une accélération identique pour tous les essais de 1400 rpm par seconde.

1.1.2.- Couche cellulose/xyloglucane

Les couches cellulose/xyloglucane ont pour objectif de détecter des activités cellulolytiques. En effet, le xyloglucane et la cellulose sont sensibles aux cellulases (ils ont le même squelette).
La tranche de silicium est placée dans le dispositif pour enduction centrifuge.
Le PAH (0.5 g/L) est déposé au centre d'une tranche de silicium, et laissé au repos pendant 5 min.
La tranche de silicium est mise en rotation pendant 1 minute à la vitesse choisie, puis est rincé à l'eau pendant 1 min.
Ensuite, la solution de cellulose/xyloglucane contenant 5 g/L de nanocristaux de cellulose et 5 g/L de xyloglucane est déposée au centre, puis laissée au repos pendant 5 min. Le xyloglucane et des nanocristaux de cellulose sont capables d'établir des interactions non covalentes, permettant de générer un réseau apte à immobiliser le mélange après séchage.
La tranche de silicium est ensuite mise en rotation pendant 3 min, puis rincé pendant 3 min.
Ces étapes sont répétées une deuxième fois pour obtenir un film à deux paires de sous-couches (une sous-couche PAH - une sous-couche d'un mélange cellulose/xyloglucane).

1.1.3.- Films d'arabinoxylane (AX)

Les films d'arabinoxylane ont été réalisés soit avec résine (10 et 15 g/L d'AX), soit sans résine (5, 10 et 15 g/L d'AX).
La préparation d'une solution d'arabinoxylane à 15 g/L avec 5% de résine, par exemple, est réalisée de la manière suivante : 3,75 mL d'arabinoxylane à 20 g/L sont prélevés, auxquels sont ajoutés 37,5 µL de résine à 10% ; le mélange est complété à 5 mL avec de l'eau ultrapure. Le volume de résine ajouté est modifié en fonction de la concentration désirée (5, 10, 15 et 20%).
Les solutions contenant 10 g/L d'arabinoxylane sont réalisées à partir de solutions à 15 g/L suivant le principe décrit ci-dessus.
En pratique, la solution de PAH est déposée au centre d'une tranche de silicium, et laissée pendant 5 min au repos. Ensuite la tranche de silicium est mise en rotation pendant 3 min, puis rincée à l'eau pendant 3 min. Pour finir la solution d'arabinoxylane est déposée au centre, laissée au repos pendant 5 min et mise en rotation pendant 3 min.

1.2.- Mesure des épaisseurs par interférométrie

Pour mesurer l'épaisseur des surfaces déposées sur les tranches de silicium, on utilise un spectrophotomètre UV-visible Specord S600.
La tranche de silicium est déposée sur un support devant deux miroirs. La lumière est réfléchie sur un premier miroir pour être ensuite transmise à l'échantillon, puis réfléchie vers le deuxième miroir et retransmise au détecteur.
La mesure de l'épaisseur du film par la lumière réfléchie est une technique bien établie, basée sur l'interaction du film avec la lumière. Elle dépend d'interférences qui résultent d'une réflexion et d'une transmission partielles aux différentes interfaces.
Les spectres sont enregistrés sur toute la gamme de longueurs d'onde couverte par le spectrophotomètre (de 182 nm jusqu'à 1019 nm).
L'acquisition des spectres et le calcul des épaisseurs de films sont réalisés avec le logiciel WinAspect.

1.3.- Tests de stabilité

Tout d'abord, la tranche de silicium, sur laquelle est déposé le polymère, est plongée dans l'eau puis séchée.

Dans un deuxième temps, la résistance de la couche de détection est testée à l'aide (i) d'une solution saline pour évaluer l'effet de la force ionique (NaCl à 0,1 mol/L), et (ii) de solutions acide et basique (HCl de pH 2 et NaOH de pH 12) pour évaluer l'effet du pH.

Une goutte de chaque solution est déposée sur le substrat, et laissée pendant trois minutes.

Le dispositif est rincé à l'eau, puis séché.

Les couches de détection sont prises en photo à chaque étape du test de stabilité.

Résultats : Stabilité des couches de détection

Pour pouvoir être efficace, les couches minces doivent être homogènes et stables en l'absence d'enzyme. Il faut aussi que les couches de détection puissent être rincées à l'eau et soient stables vis à vis des solutions aqueuses étudiées.

Deux cas sont distingués :

- les mélanges cellulose/xyloglucane : les films sont stables sans ajout de réticulant ; nous avons donc testé uniquement l'effet de la force ionique et du pH sur les couches minces.
- Les arabinoxylanes : les films ne sont pas stables seuls (un simple lavage à l'eau suffit pour faire disparaître la couleur car la cohésion des chaînes de polymères au sein des couches est faible).

L'ajout de résine formaldéhyde/urée a été testé pour stabiliser la couche de détection à base d'arabinoxylane vis à vis du pH et de la force ionique ; la quantité de résine nécessaire a ensuite été optimisée.

2.1.- Cellulose / Xyloglucane

Lors du test de stabilité des gouttes de NaCl (0,1 mol/L), HCl (pH 2) et NaOH (pH 12) sont chacune déposées sur la couche de détection, et laissé pendant trois minutes ; puis la couche de détection est rincée à l'eau.

On observe que la surface ne change pas de couleur, avant et après le test.

Par conséquent la bicouche de cellulose et de xyloglucane est stable envers les milieux testés.

2.2.- Arabinoxylane

L'influence de plusieurs paramètres a été étudiée sur la stabilité des couches d'arabinoxylane : le type d'arabinoxylane (haute viscosité (HV) et moyenne viscosité (MV)), la concentration (15g/L et 10g/L) et le pourcentage de résine de réticulation présente dans la solution d'arabinoxylane (entre 5 et 20%).

2.2.1.- Effet du type d'arabinoxylane

Avant de faire le test de stabilité dans des conditions agressives, les couches ont été baignées dans l'eau.

On étudie pour cela des couches d'arabinoxylane moyenne viscosité à 15g/L et 10g/L avec de la résine à 20%. Après un bain dans l'eau, on observe que ces couches restent homogènes.

On étudie ensuite des couches d'arabinoxylane haute viscosité à 15g/L et 10g/L avec 20% de résine. Après le bain dans l'eau, on observe un changement de couleur qui peut être dû à une diminution de l'épaisseur et lié à une perte de polymère. De plus, on observe que ces couches sont moins homogènes que les couches d'arabinoxylane moyenne viscosité.

Les couches d'arabinoxylane haute viscosité ne sont donc pas stables. Cela peut être lié au fait que les molécules forment des agrégats, la masse moléculaire de l'arabinoxylane étant élevée. La résine peut donc difficilement réticuler.

2.2.2.- Effet de la concentration et du pourcentage de résine sur la stabilité des couches

A l'issu des résultats précédents, des tests sont mis en oeuvre sur des couches d'arabinoxylane moyenne viscosité construites à une vitesse de rotation de 2000rpm et à partir de solutions de concentrations de 10 et 15 g/L.

Pour déterminer la stabilité de la couche, on a utilisé deux paramètres : la force ionique et le pH (acide et basique).

Des solutions de NaCl à 0,1mol/L, de HCl à pH2 et de NaOH à pH12 ont été déposées sur les couches minces de la même façon.

Comme la xylanase est diluée dans du tampon acétate, il faut vérifier si le tampon dégrade la couche de polymère. Ce tampon acétate est donc également testé sur les couches d'arabinoxylane.

Les tests de stabilité ont été réalisés sur les films d'arabinoxylane moyenne viscosité à 15g/L et 10g/L, pour différentes concentrations de résine (5%, 10%, 15% et 20%).

On observe un changement de couleur à l'emplacement des dépôts, en particulier pour des concentrations de résine de 5%, 10%, 15%. Ce changement de couleur est moins visible lorsque le pourcentage de résine augmente.

Pour la suite de l'étude, les couches qui nous intéressent doivent être suffisamment stables pour ne pas être rincés en solution mais doivent permettre de visualiser une activité enzymatique.

Pour répondre à ces deux critères, on choisit avantageusement les couches contenant 15% de résine.

Les couches d'arabinoxylane moyenne viscosité à 15g/L sont stables pour un pourcentage de résine de 20% et les couches d'arabinoxylane à 10g/L sont stables pour les pourcentages de résine de 15 et 20%.

On observe sur les deux échantillons d'arabinoxylane moyenne viscosité à 15g/L et 10g/L, qu'il y a une tâche persistante correspondant à la solution acide, quel que soit la concentration de résine. Cela peut être dû au fait que l'arabinoxylane est hydrolysée en présence d'acide. Il faut donc prendre des précautions, et utiliser l'enzyme dans un milieu peu acide.

On remarque que le tampon acétate ne dégrade pas la couche mince. On peut donc diluer l'enzyme dans du tampon acétate à pH 5, qui est la condition optimale pour l'enzyme.

2.3.- Conclusion

La couche de cellulose/xyloglucane est stable en présence de sel, à pH acide et à pH basique.

Les couches d'arabinoxylane à haute viscosité ne sont pas homogènes après le bain dans l'eau, alors que les films d'arabinoxylane à moyenne viscosité sont stables.

Sur les couches d'arabinoxylane moyenne viscosité, on constate que les tâches sont moins visibles lorsque la concentration en résine augmente. On choisit donc de poursuivre l'étude avec les films d'arabinoxylane moyenne viscosité à 15% de résine.

Des essais similaires ont permis de mettre en évidence une stabilité également pendant 24 heures.

**Exemple 2 : Mesure de l'activité enzymatique**

La détection de l'activité enzymatique par les dispositifs issus de l'Exemple 1 ci-dessus, est comparée avec une méthode classique dite de « Nelson ».

Les enzymes étudiées ci-après consistent en :

- la β-xylanase M1 (8000 U ; 216 U/mg ; 2300 U/mL; commercialisé par Megazyme), et
- la cellulyve, issue d'un *Trichoderma Reesei* qui n'a pas été totalement purifiée (activité théorique est de 4880 nkat/g, 49 mg/g) (commercialisé par Lyven).

1.- Méthode Nelson

La méthode Nelson permet de doser les extrémités réductrices libérées lors de l'hydrolyse d'un polysaccharide par une enzyme. C'est un dosage indirect : en milieu alcalin et à ébullition, le groupement pseudo-aldéhydique des extrémités réductrices réduit les ions cuivriques en ions cuivreux. Ces derniers réagissent avec le réactif arsénio-molybdique pour donner une coloration bleue dont la densité optique (DO) varie linéairement avec la quantité d'oses réducteurs. Le nombre d'extrémités est dosé au bout de 10 minutes d'incubation.

Le dosage de Nelson se déroule en deux étapes :

- détermination d'une droite d'étalonnage qui détermine la réponse de la méthode en fonction de la concentration en extrémités réductrices ; cette étape est réalisée sur des sucres monomères (glucose et xylose).
- dosage des activités des enzymes que nous allons étudier sur des polymères en solution : nous allons mesurer les activités d'une xylanase et d'une cellulase sur les arabinoxylanes et le xyloglucane respectivement.

1.1.- Matériel

Solution A:

200 g $Na_2SO_4$ anhydre
25 g $Na_2CO_3$ anhydre
25 g Tartrate double Na-K
20 g $NaHCO_3$ anhydre

Solution B:

30 g $CuSO_4$, $5H_2O$
4 gouttes $H_2SO_4$ concentré
$H_2O$ qsp 200 mL

Solution C: 25 mL de la solution A + 1 mL de la solution B

Solution D :

50 g $(NH_4)_6$ $MO_7O_2$ $4H_2O$ dissous dans 800 mL $H_2O$
42 mL $H_2SO_4$ concentré, versés rapidement
6 g $Na_2$ $HAsO_4$, $7H_2O$ dissous dans un peu d'eau
$H_2O$ qsp 1 L

1.2.- Préparation des gammes étalon

On prépare une solution mère de l'ose à 250 µg/mL.
On utilise ici une solution mère de glucose, comme référence pour le xyloglucane. La solution mère de xylose sera la référence pour les deux solutions d'arabinoxylane à haute et moyenne viscosité.
Différentes dilutions de la solution mère sont réalisées entre 0 et 250 µg/mL. La densité optique est mesurée pour chaque concentration afin d'obtenir une gamme étalon.

1.3.- Dosage des activités enzymatiques :

*Réaction enzymatique*

Pour la préparation des échantillons, 135 µL de la solution mère et 15 µL de solution enzymatique sont mis dans un tube et incubés à 40°C pendant dix minutes.
Au bout de 10 minutes, l'activité enzymatique est arrêtée par l'ajout de 100 µL d'un échantillon dans 100 µL de solution C.
La préparation du blanc permet de mesurer la DO lorsque l'enzyme est désactivée. Le blanc est composé de 100 µL de la solution C, auquel on ajoute 10 µL d'enzyme et 90 µL de la solution mère.

*Réaction colorimétrique*

Les échantillons sont mis 15 min au bain marie bouillant.
Les échantillons sont ensuite refroidis et la solution D est ajoutée (100 µL) permettant d'obtenir des couleurs différentes entre le blanc et les échantillons.
Les plaques sont maintenues sous agitation pendant dix minutes, puis 1 mL d'eau est ajouté.
Pour finir 250 µL de chaque puit est transvasé dans une plaque 96 puits, pour lecture de la densité optique sur un lecteur de plaque à 600 nm.
Pour exprimer les résultats, la concentration de la gamme étalon doit être exprimée en µmol.
La formule (1) suivante est utilisée pour le calcul de l'activité :

$$a = DeltaDO / pente \times 150 / 15 \times 1000 / t \times dil \qquad (1)$$

avec : a : activité enzymatique en nkat/mL
DeltaDO : différence de densité optique entre l'échantillon et le blanc

La pente est exprimée en µmol

150 est le volume total de milieu réactionnel
15 est le volume de prise d'essai d'enzyme
t est le temps d'incubation en s (pour cette expérience t = 600 s)
dil est la dilution de l'enzyme à laquelle la réaction enzymatique a été faite

La variation de DO minimum acceptable pour que la mesure soit valable est de 0,1.
La formule (2) utilisée pour calculer l'activité minimale est la suivante :

$$Delta(a) = Delta(DeltaDO) / pente \times 150 / 15 \times 1000 / t \qquad (2)$$

Le calcul a été fait pour les gammes étalon du xylose et du glucose.
A partir ces résultats, on déduit que la plus faible activité mesurable par cette méthode est de 3 nkat/mL.

2.- Evaluation de l'activité enzymatique sur couche de détection

Une goutte de la solution enzymatique à tester et une goutte du témoin (1 mL de la solution enzymatique diluée chauffée à 110°C pendant une heure) sont déposées sur la couche de détection pendant trois minutes.
Les dispositifs selon l'invention sont rincés à l'eau et séchés.
Les couches de détection sont prises en photo à chaque étape du test enzymatique.
Le premier test se fait avec une solution enzymatique à 125 µg/mL (0.6nkat/mL) pour la cellulyve, et la solution est diluée au 2000ème pour la xylanase (19nkat/mL).
Ensuite nous diluons les solutions enzymatiques jusqu'à ce que l'activité enzymatique ne soit plus visible sur les surfaces.

3.- Résultats

3.1.- Méthode Nelson

*Droite d'étalonnage*

Tout d'abord nous avons fait deux gammes étalons, qui permettent de déterminer la relation entre la densité optique mesurée et la concentration en extrémités réductrices.
La gamme étalon du glucose est la référence pour le xyloglucane ; la gamme étalon du xylose est la référence pour l'arabinoxylane.
Les droites d'étalonnage ont permis de calculer l'activité de l'enzyme en déterminant le nombre d'extrémités réductrices libérés (donc les coupures effectuées par l'enzyme) dans un temps déterminé.

*Mesure de l'activité de la Cellulyve*

Nous avons étudié l'activité de la Cellulyve sur le xyloglucane. La cellulyve est issue d'un *Trichoderma Reesei,* champignon qui est capable de secréter une grande quantité d'enzymes cellulosiques.
Nous avons préparé une solution mère de cellulyve à 0,5mg/mL dont l'activité théorique est d'environ 2,4nkat/mL.
A partir des droites d'étalonnage, on détermine une activité enzymatique de 7nkat/mL. Ce résultat est légèrement différent de l'activité théorique, mais du même ordre de grandeur.

*Mesure de l'activité de la xylanase*

Nous avons vérifié l'activité de la xylanase sur deux substrats : les arabinoxylanes haute et moyenne viscosité.
L'activité de la xylanase pure, présentée par le fournisseur, est estimée à environ 38 000 nkat/mL.
Nous avons calculé l'activité de la xylanase pure à partir des résultats obtenus pour des solutions diluées au 2000ème et au 1000ème.
Les résultats obtenus sont présentés dans le tableau 1 ci-dessous.

Tableau 1

|  | Activité de la xylanase mesurée nkat/mL | Activité de la xylanase pure nkat/mL |
|---|---|---|
| AX-HV | | |
| Dilution au 1000ème | 32 | 31 889 |
| Dilution au 2000ème | 16 | 31 943 |
| AX-MV | | |
| Dilution au 1000ème | 33 | 32 828 |
| Dilution au 2000ème | 19 | 37 080 |

On retrouve une valeur d'environ 32 000 nkat/mL, qui est du même ordre de grandeur que la valeur donnée par le fournisseur.

3.2.- Activité enzymatique sur couches de détection

*Cellulose/Xyloglucane*

Comme développé ci-dessus dans l'Exemple 1, la couche de détection cellulose/xyloglucane est formée par des sous-couches d'un mélange nanocristaux de cellulose de coton (Ct) et de xyloglucane (XG), déposées en alternance avec des sous-couches de chlorhydrate de poly(allylamine) (PAH).

La couche obtenue présente une épaisseur totale de 175 nm et une couleur jaune pâle.

Dans un premier temps, on se place à la même concentration de Cellulyve que celle utilisée pour la méthode de Nelson (125$\mu$g/mL, soit 0,6 nkat/mL).

Une goutte de solution enzymatique est déposée (0,6 nkat/mL) et une goutte d'enzyme inactivée est déposée (témoin : 1 mL de la solution d'enzyme diluée chauffée à 110°C pendant une heure).

Après dépôt d'une goutte de cellulase, rinçage et séchage, une couleur bleu apparaît à l'endroit où l'enzyme a été déposée, laissant supposer la diminution de l'épaisseur de la couche.

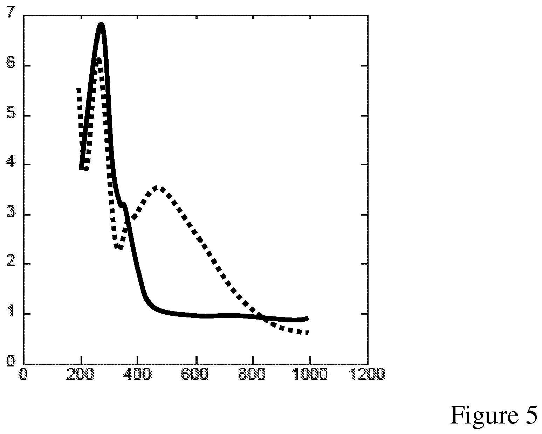

Cette diminution d'épaisseur a été confirmée par interférométrie et par microscopie à force atomique. La mesure du profil d'interférence (figure 5), avant et après action de l'enzyme, montre sans ambiguïté que le phénomène d'interférence présent à l'origine (avant l'action de l'enzyme) a disparu après l'action de l'enzyme. Ce résultat indique que l'épaisseur de la couche de détection est inférieure à 150 nm.

Alors que la couche reste inchangée après un lavage à l'eau, son épaisseur est fortement diminuée après action de l'enzyme. Il faut noter que la rugosité de la couche après action de l'enzyme reste remarquablement faible. Il semble que, sans être lié par aucune théorie, (i) soit l'enzyme procède par érosion de la couche sans pénétrer la structure, (ii) soit la PAH forme une barrière à l'action de l'enzyme.

On voit ici que ce dispositif est susceptible de constituer une opportunité pour étudier l'action d'enzyme face à des substrats solides. Ce dispositif devrait ainsi permettre de reconstituer un système modèle en milieu hétérogène.

Ensuite l'enzyme est diluée plusieurs fois pour déterminer le seuil de détection de l'activité enzymatique. Les solutions étudiées sont présentées dans le tableau 2 suivant.

Tableau 2

| Nom de solutions | Dilution | Activité enzymatique |
|---|---|---|
| S1 | 1/40ème | 0,06 nkat/mL |
| S2 | 1/400ème | $6.10^{-3}$ nkat/mL |
| S3 | 1/4000ème | $6.10^{-4}$ nkat/mL |
| S4 | 1/40 000ème | $6.10^{-5}$ nkat/mL |

En pratique, on observe que plus on dilue (de S1 à S4), moins le changement de couleur est significatif par rapport à la couleur d'origine. Ainsi, plus on dilue, moins le film de polymère est dégradé.

L'activité minimale que l'on peut détecter visuellement, au bout de 3 min, est de 0,06nkat/mL (S1).

Or la méthode de Nelson permet de détecter une activité minimale de 3 nkat/mL. Les tests sur le dispositif selon l'invention sont donc 20 fois plus sensibles que la méthode de Nelson.

Par ailleurs la méthode de Nelson nécessite un temps d'incubation de 10 minutes alors que le test selon l'invention dure 3 minutes. Ceci prouve aussi que la méthode selon l'invention est plus rapide.

*Arabinoxylane (HV-MV)*

Là encore, on se place à la même concentration d'enzyme que celle de la méthode de Nelson (xylanase diluée au 1/2000ème soit 19nkat/mL)

On dépose une goutte de la solution enzymatique (19nkat/mL) et une enzyme inactivée (témoin) sur les films d'arabinoxylane à 15g/L (couleur bleu) et 10g/L (couleur marron gris).

On peut alors observer, après incubation et rinçage, que la couleur disparaît là où l'enzyme a été déposée ; alors que la couleur reste inchangée sur le site témoin.

Comme pour les couches à base de cellulose et de xyloglucane, on détecte donc effectivement une activité enzymatique.

Pour l'arabinoxylane à 10g/L, l'activité enzymatique est moins visible car la couleur initiale de la couche de détection (marron gris) est assez proche de celle de la tranche de silicium. Il y a ainsi peu, voire pas, de changement au niveau de la tonalité chromatique.

Ensuite, d'autres tests sont réalisés avec une dilution de l'enzyme pour déterminer le seuil minimal de détection. Les solutions étudiées sont présentées dans le tableau 3 ci-dessous.

Tableau 3

| Nom de solutions | Dilution | Activité enzymatique |
|---|---|---|
| S1 | 20 000ème | 1,9 nkat/mL |
| S2 | 200 000ème | 0,19 nkat/mL |
| S3 | $2.10^6$ème | $1.9\ 10^{-2}$ nkat/mL |
| S4 | $2.10^7$ème | $1.9\ 10^{-3}$ nkat/mL |

Tout d'abord, on étudie les différentes dilutions de la xylanase qui ont agit sur les films d'arabinoxylane moyenne viscosité à 15g/L et 10g/L.

Sur le film d'arabinoxylane moyenne viscosité à 15g/L, la couleur disparaît pour une solution enzymatique d'activité 1,9 nkat/mL (S1). On distingue encore un changement de couleur pour une activité enzymatique jusqu'à $1,9.10^{-2}$ nkat/mL (S3).

Le test sur les surfaces d'arabinoxylane est donc 150 fois plus sensible que la méthode de Nelson.

L'épaisseur des films d'arabinoxylane moyenne viscosité à 10g/L est inférieure à celle des films d'arabinoxylane à 15g/L; et les enzymes dégradent plus facilement les films d'arabinoxylane à 10g/L.

Pour les films d'arabinoxylane moyenne viscosité à 10g/L, le changement de couleur est moins visible que sur les films d'arabinoxylane à 15g/L. Cela vient encore du fait que l'épaisseur des films d'arabinoxylane à 10g/L est plus faible et présente une couleur qui est plus proche de celle de la tranche de silicium.

4.- Conclusion

Les activités des enzymes déterminées par la méthode de Nelson correspondent aux activités données par les fournisseurs.

Pour le test sur les dispositifs selon l'invention, on a utilisé les mêmes concentrations d'enzymes que celles utilisées pour la méthode de Nelson. Une activité enzymatique a effectivement pu être observée.

Des dilutions ont été réalisées pour déterminer le seuil de détection d'une activité enzymatique au moyen du dispositif selon l'invention.

Les résultats obtenus montrent que le dispositif selon l'invention permet de détecter des activités enzymatiques 20 à 150 fois plus faibles que celles mesurées par la méthode de Nelson, et ce en trois fois moins de temps.

**Exemple 3 : Détection d'activité enzymatique**

Matériel

Des essais complémentaires de détection d'activité enzymatique ont été mis en oeuvre sur des dispositifs comportant une couche de détection déposée selon un protocole détaillé dans l'Exemple 1.

Un premier dispositif utilisé, désigné ci-après (PAH-C5/XG1)$_4$, comporte une couche de détection de couleur bleu, constituée de 4 paires de sous-couches, chaque paire de sous-couches étant constituée :

- d'une sous-couche de PAH déposée à une concentration de 0,5g/L, et
- d'une sous-couche constituée d'un mélange de nanocristaux de cellulose et de xyloglucane, déposé respectivement à une concentration de 5g/L et de 1g/L.

La présente couche de détection a une épaisseur initiale estimée à 160 nm.

Un second dispositif utilisé, désigné ci-après (C5-XG1)$_8$, comporte également une couche de détection de couleur bleu, constituée de 8 paires de sous-couches, chaque paire de sous-couches étant constituée :

- d'une sous-couche de nanocristaux de cellulose, déposée à une concentration de 5g/L, et
- d'une sous-couche de xyloglucane, déposée à une concentration de 1 g/L.

La présente couche de détection a une épaisseur initiale estimée à 90 nm.

L'enzyme étudiée consiste en un mélange d'enzymes Cellulyve TR de la société Lyven, composé d'un mélange de plusieurs cellulases, dont l'activité initiale est de 4880 nkat/g.

Cette solution d'enzymes est testée à différentes concentrations sur ces deux dispositifs, respectivement 0,6 nkat/mL, 0,24 nkat/mL, 0,12 nkat/mL, 0,06 nkat/mL, 0,024 nkat/mL et 0,006 nkat/mL.

Résultat

Les gouttes des solutions enzymatiques aux différentes concentrations sont déposées sur les couches de détection, qui sont mises à l'étuve à 50°C pendant 3, 5, 10 et 15 min.

L'aspect des films (C5-XG1)$_8$ et (PAH-C5/XG1)$_4$ est comparé avant et après le test de sensibilité enzymatique. Une activité est détectée lorsque la couleur de la couche de détection est modifiée.

Pour les échantillons (C5-XG1)$_8$, on peut observer aux différents temps :

- à 3 min, les activités détectées sont celles à 0,6, 0,24 et 0,12 nkat/mL ; la surface de la tranche de silicium est visible pour 0,6 nkat/mL.
- à 5 min, l'activité à 0,06 nkat/mL est discernable, et la surface du substrat est atteinte pour les activités à 0,6 et 0,24 nkat/mL.
- à 10 min, l'activité à 0,06 nkat/mL est distinctement visible, et la surface est atteinte pour les trois premières concentrations.
- à 15 min, l'activité enzymatique est détectée pour toutes les concentrations, rendant la surface du wafer visible pour les 4 premières concentrations ; l'activité à 0,024 nkat/mL forme une tâche bleue.

Pour les échantillons (PAH-C5/XG1)$_4$, on peut observer aux différents temps d'incubation :

- à 3 min, les activités détectées sont celles à 0,6 et 0,24 nkat/mL.
- à 5 min, l'activité à 0,12 nkat/mL est également détectée.
- à 10 min, on discerne aussi l'activité à 0,024 nkat/mL.
- à 15 min, l'attaque à une concentration de 0,024 nkat/mL est toujours visible.

Les tests visuels montrent clairement que l'action des enzymes est fonction du temps.

En effet, pour les échantillons (C5-XG1)$_8$, à de fortes activités enzymatiques ou à des activités plus faibles mais pour des temps d'incubation plus long, la surface de la tranche de silicium est visible.

De plus, des tâches de couleurs intermédiaires (bleu clair ou bleu fonce) apparaissent lorsque les temps et/ou concentrations ne permettent pas une attaque jusqu'à la tranche de silicium.

Pour les échantillons (PAH-C5/XG1)$_4$, à toutes les concentrations et temps, la coloration obtenue après l'attaque enzymatique est un bleu, plus ou moins intense.

L'attaque se fait donc sur toute l'épaisseur des films (C5-XG1)$_8$, faisant apparaitre la surface des tranches de silicium dès 3 min. Toutefois, pour les échantillons (PAH-C5/XG1)$_4$, les attaques semblent être stoppées à un même niveau de profondeur, la couleur bleu foncée correspondant a une épaisseur de 120 nm, ce qui équivaut à 3,5 bicouches de PAH-C5/XG1, soit l'épaisseur après le dépôt de PAH.

D'après ces résultats et sans être liés par aucune théorie, il semble possible que l'attaque des enzymes sur les films (PAH-C5/XG1)$_4$ soit stoppée par la couche de PAH. Le PAH jouerait donc un rôle de barrière aux enzymes, qui ne s'attaquent qu'à la cellulose et au xyloglucane, et ne permet donc pas d'atteindre la surface des tranches de silicium comme pour les films (C5-XG1)$_8$.

Ces constructions présentent chacune des avantages.

La construction PAH-C5/XG1 permet un choix des couleurs souhaitées en fin de test, en faisant varier son épaisseur. Toutefois, elle ne permettra pas de quantifier « l'intensité » d'attaque, les couleurs finales étant les mêmes.

La construction C5-XG1 permet de voir de façon plus franche l'attaque enzymatique. En effet, la totalité de la couche de détection peut être dégradée quand les conditions sont réunies, et permet de voir des attaques non-complètes, de par un changement de couleurs de la couche de détection.

Dans les deux cas de figure, la détection est 50 fois plus sensible que lors des essais selon l'Exemple 2.

**Exemple 4 : Détection d'activité enzymatique**

Matériel

Des essais complémentaires de détection d'activité enzymatique ont été mis en oeuvre sur des dispositifs comportant une couche de détection déposée selon un protocole détaillé dans l'Exemple 1.

Deux dispositifs utilisés, désignés ci-après (C5-XG1)$_5$ et (C5-XG1)$_8$, comportent une couche de détection constituée, respectivement, de 5 et de 8 paires de sous-couches.

Chaque paire de sous-couches est constituée :

- d'une sous-couche de nanocristaux de cellulose, déposée à une concentration de 5g/L, et
- d'une sous-couche de xyloglucane, déposée à une concentration de 1 g/L.

Les films présentent respectivement :

- pour le film (C5-XG1)$_5$, une couleur marron et une épaisseur de l'ordre de 70 nm, et
- pour le film (C5-XG1)$_8$, une couleur bleu et une épaisseur de l'ordre de 127,5 nm avec une variation standard de 3,28 nm.

L'enzyme étudiée consiste en un mélange d'enzymes Cellulyve TR de la société Lyven, composé d'un mélange de plusieurs cellulases, dont l'activité initiale est de 4880 nkat/g.

Cette solution d'enzymes est testée sur ces deux dispositifs aux concentrations respectivement suivantes : 100 mg/L, 50 mg/L, 25 mg/L, 20 mg/L, 15 mg/L, 10 mg/L, 1 mg/L et 0,1 mg/L.

Résultat

Des gouttes de $2\mu$L des solutions enzymatiques, aux différentes concentrations, sont déposées sur les couches de détection qui sont mises à l'étuve à 50°C pendant 10 min, 30 min et 60 min.

L'aspect des films (C5-XG1)$_5$ et (C5-XG1)$_8$ est comparé avant et après le test de sensibilité enzymatique. Une activité est détectée lorsque la couleur de la couche de détection est modifiée.

Pour l'échantillon (C5-XG1)$_8$, après 10 min de réaction, le substrat est visible pour les concentrations de 100 mg/L et de 50 mg/L, ce qui suggère une dégradation totale du film.

De plus, la couleur du film tourne vers le brun pour certaines des concentrations inférieures suivantes, à savoir de 25 mg/L, 20 mg/L et 15 mg/L, alors qu'elle demeure inchangée à partir d'une concentration de 10 mg/L.

Après 30 min de réaction, la dégradation de l'échantillon (C5-XG1)$_8$, par les solutions d'enzymes aux concentrations 15 et 10 mg/L, est clairement visible.

L'augmentation du temps de réaction à 60 min n'améliore pas significativement la détection, en ce sens que les concentrations les plus faibles (1 mg/L et 0,1 mg/L) n'entrainent toujours pas de changement de couleur.

En revanche, il est intéressant de noter que la dégradation par la solution d'enzymes à la concentration 10 mg/L est accrue à 60 min d'incubation, en comparaison d'un temps d'incubation de 30 min, puisque la couleur marron visible à 30 min est remplacée par la couleur grise du substrat à 60 min.

Les essais sur l'échantillon (C5-XG1)$_5$ montrent des caractéristiques similaires.

La seule différence porte sur la solution d'enzymes à la concentration 10 mg/L : la dégradation est complète pour l'échantillon (C5-XG1)$_5$ après 30 min, alors que cette dégradation était partielle pour l'échantillon (C5-XG1)$_8$ comme précisé ci-dessus.

Ces résultats montrent que le temps de réaction et l'épaisseur du film constituent des paramètres importants pour optimiser la sensibilité et la réponse lors de l'essai.

De plus, la combinaison du temps de réaction et de l'épaisseur de la couche de détection constitue des paramètres ajustables pour une évaluation semi-quantitative de l'activité enzymatique.

Par exemple, la solution d'enzymes dont la concentration est 10 mg/L n'est pas détectée après 10 min d'incubation, mais présente un résultat positif après 30 min d'incubation (pour rappel, on obtient une dégradation partielle pour l'échantillon (C5-XG1)$_8$).

**Exemple 5 : Formulation et de dégradation pour des biopolymères supplémentaires**

Afin d'évaluer les possibilités d'application de la méthode selon l'invention à d'autres biopolymères, nous avons réalisé une évaluation sommaire de plusieurs couples biopolymères/enzymes en utilisant la disparition de la couleur comme validation de la détection de l'activité.

Conformément aux Exemples 1 à 3, les dépôts ont été réalisés sur des substrats de silicium possédant un indice de réfraction plus élevé que celui de la couche de biopolymères, permettant d'exacerber les couleurs.

Dans chaque cas, nous avons utilisé de l'eau et la solution d'enzyme désactivée thermiquement (la solution est portée à ébullition pendant quelques minutes puis déposées) comme références.

Pour tous les résultats présentés ci-après, les solutions d'enzyme, les solutions d'enzyme désactivée et d'eau, ont été incubées durant 3 min à température ambiante.

Dépôt Sérum albumine bovine dégradation par la trypsine.

La couche de Sérum albumine bovine a été obtenue par « spin-coating » d'une solution 10 g/L contenant 0,1 g/L d'une résine MUF. La couche a été réticulée à 90°C pendant une heure.

Dépôt Sérum albumine bovine en mélange avec du chitosan, dégradation par la trypsine.

La couche de Sérum albumine bovine a été obtenue par « spin-coating » d'une solution 10 g/L de SAB mélangé à du chitosan (10g/L) pour favoriser la réticulation. La couche a été immobilisée en ajoutant la solution 0,1 g/L d'une résine MUF suivi d'une réticulation à 90°C pendant une heure.

Dépôt Pectine et dégradation par un cocktail pectinolvtiaue.

La couche de pectine a été obtenue par « spin-coating » d'une solution 10 g/L contenant 5% en poids de résine MUF. La couche a été réticulée à 90°C pendant une heure.

Résultats

Dans les trois cas, après incubation et rinçage, on observe un changement de couleur uniquement au niveau du site de dépôt de la solution d'enzyme active.

**Revendications**

**1.** Dispositif colorimétrique pour la détection, dans une solution aqueuse d'intérêt, d'une activité enzymatique hydrolytique à l'égard d'au moins un polymère d'intérêt,
lequel dispositif (1) est **caractérisé en ce qu'**il comprend (i) un substrat (2) délimité au moins par une surface supérieure (2a), et (ii) une couche transparente de détection (3), ayant une première épaisseur e et comprenant ledit polymère d'intérêt,
laquelle couche transparente de détection (3) comporte, d'une part, une surface supérieure (3a), sur laquelle est destinée à être appliquée ladite solution aqueuse d'intérêt et formant une première interface (4) entre l'air et la couche de détection (3), et d'autre part, une surface inférieure (3b), formée sur ladite surface supérieure (2a) du substrat (2) et formant ensemble une seconde interface (5) entre la couche de détection (3) et le substrat (2),
laquelle surface supérieure (3a) de la couche transparente de détection (3) est libre, étant aucunement recouverte par une couche additionnelle,
laquelle première interface (4) et laquelle seconde interface (5) sont aptes à générer un phénomène optique de réflexion,
laquelle couche de détection (3) est adaptée pour, d'une part, après l'application de ladite solution aqueuse d'intérêt lorsque celle-ci est dépourvue de ladite activité enzymatique hydrolytique, conserver ladite première épaisseur e, et d'autre part, suite à l'application de ladite solution aqueuse d'intérêt lorsque celle-ci comporte ladite activité enzymatique hydrolytique, présenter une seconde épaisseur e', inférieure à ladite première épaisseur e,
et **en ce que** ladite première épaisseur e et/ou ladite seconde épaisseur e' de ladite couche transparente de détection (3) sont adaptées pour engendrer une couleur par un phénomène optique d'interférence généré par la recombinaison des faisceaux lumineux réfléchis à ladite première interface (4) et à ladite seconde interface (5).

2. Dispositif colorimétrique selon la revendication 1, **caractérisé en ce que** la première épaisseur e de la couche de détection (3) est choisie pour engendrer une première couleur par un phénomène optique d'interférence, et **en ce que** ladite couche de détection (3) est adaptée pour, d'une part, après l'application de ladite solution aqueuse d'intérêt lorsque celle-ci est dépourvue de ladite activité enzymatique hydrolytique, conserver ladite première épaisseur e engendrant ladite première couleur, et d'autre part, suite à l'application de ladite solution aqueuse d'intérêt lorsque celle-ci comporte ladite activité enzymatique hydrolytique, présenter une seconde épaisseur e', inférieure à ladite première épaisseur e, engendrant soit une seconde couleur par un phénomène optique d'interférence, différente de ladite première couleur, soit la disparition de ladite première couleur.

3. Dispositif colorimétrique selon l'une quelconque des revendications 1 ou 2, **caractérisé en ce que** la première épaisseur e de la couche transparente de détection (3) est une épaisseur uniforme, choisie dans un domaine d'épaisseur compris entre 70 et 900 nm.

4. Dispositif colorimétrique selon l'une quelconque des revendications 1 à 3, **caractérisé en ce que** la première épaisseur e et/ou la seconde épaisseur e' de ladite couche transparente de détection (3) sont adaptées pour engendrer une couleur par un phénomène optique d'interférence, avec une réflectance maximale pour une longueur d'onde comprise entre 380 nm et 780 nm.

5. Dispositif colorimétrique selon l'une quelconque des revendications 1 à 4, **caractérisé en ce que** la couche transparente de détection (3) a un indice de réfraction $n_c$ compris entre 1,2 et 1,7, et de préférence compris entre 1,4 et 1,6.

6. Dispositif colorimétrique selon l'une quelconque des revendications 1 à 5, **caractérisé en ce que** le polymère contenu dans la couche transparente de détection (3) est choisi parmi les biopolymères.

7. Dispositif colorimétrique selon l'une quelconque des revendications 1 à 6, **caractérisé en ce que** le substrat (2) est transparent, et **en ce que** la couche transparente de détection (3) a un indice de réfraction $n_c$ qui est différent de l'indice de réfraction $n_s$ dudit substrat (2).

8. Dispositif colorimétrique selon l'une quelconque des revendications 1 à 6, **caractérisé en ce que** le substrat (2) est opaque et comporte une surface supérieure (2a) réfléchissante.

9. Dispositif colorimétrique selon l'une quelconque des revendications 1 à 8, **caractérisé en ce que** le ou les polymères contenus dans la couche transparente de détection (3) sont sous une forme immobilisée.

10. Dispositif colorimétrique selon la revendication 9, **caractérisé en ce que** la couche transparente de détection (3) contient des nanocristaux formant des réseaux de liaisons hydrogènes avec le polymère d'intérêt.

11. Dispositif colorimétrique selon la revendication 10, **caractérisé en ce que** les nanocristaux consistent en des nanocristaux de polysaccharides, et de préférence en des nanocristaux de cellulose.

12. Dispositif colorimétrique selon l'une quelconque des revendications 10 ou 11, **caractérisé en ce que** les nanocristaux ont une charge de surface négative, **en ce que** la couche transparente de détection (3) est constituée d'au moins deux sous-couches (6) contenant chacune le polymère d'intérêt, et **en ce que** lesdites sous-couches de polymère (6) sont séparées deux à deux par une sous-couche intercalaire (7) contenant un composé poly-cationique.

13. Dispositif colorimétrique selon l'une quelconque des revendications 10 ou 11, **caractérisé en ce que** la couche transparente de détection (3) est constituée d'au moins une paire de deux sous-couches (6, 7) contenant chacune au moins un polymère d'intérêt, avec une première sous-couche (6) comportant un premier ou des premiers polymères d'intérêt, et une seconde sous-couches (7) contenant un second ou des seconds polymères d'intérêt.

14. Dispositif colorimétrique selon la revendication 9, **caractérisé en ce que** la couche transparente de détection (3) contient une résine formant des réseaux de liaisons covalentes avec le polymère d'intérêt.

15. Procédé pour la détection, dans une solution aqueuse d'intérêt, d'une activité enzymatique hydrolytique à l'égard d'au moins un polymère d'intérêt, **caractérisé en ce qu'**il comprend au moins la succession d'étapes suivantes :

- la fourniture d'un dispositif colorimétrique (1) selon l'une quelconque des revendications 1 à 14,
- l'application de ladite solution aqueuse d'intérêt sur la surface supérieure (3a) de la couche transparente de détection (3),
- le lavage de ladite surface supérieure (3a) de la couche transparente de détection (3),
- le séchage de ladite couche transparente de détection (3), et
- l'analyse de la couleur de ladite couche transparente de détection (3) au niveau du site d'application de ladite solution d'intérêt.

**Patentansprüche**

1. Kolorimetrische Vorrichtung zum Nachweis, in einer wässrigen Lösung von Interesse, einer hydrolytischen Enzymaktivität gegenüber mindestens einem Polymer von Interesse,
   wobei die Vorrichtung (1) **dadurch gekennzeichnet ist, dass** sie (i) ein Substrat (2), das durch mindestens eine obere Oberfläche (2a) abgegrenzt ist, und (ii) eine transparente Nachweisschicht (3) umfasst, die eine erste Dicke e aufweist und das Polymer von Interesse umfasst,
   wobei die transparente Nachweisschicht (3) einerseits eine obere Oberfläche (3a) enthält, auf die die wässrige Lösung von Interesse aufgebracht werden soll und die eine erste Grenzfläche (4) zwischen der Luft und der Nachweisschicht (3) bildet, und andererseits eine untere Oberfläche (3b), die auf der oberen Oberfläche (2a) des Substrats (2) gebildet wird und die zusammen eine zweite Grenzfläche (5) zwischen der Nachweisschicht (3) und dem Substrat (2) bilden,
   wobei die obere Oberfläche (3a) der transparenten Nachweisschicht (3) frei ist, in keiner Weise durch eine weitere Schicht bedeckt ist,
   wobei die erste Grenzfläche (4) und die zweite Grenzfläche (5) dafür ausgelegt sind, ein optisches Reflexionsphänomen zu erzeugen,
   wobei die Nachweisschicht (3) einerseits dafür ausgelegt ist, nach dem Aufbringen der wässrigen Lötung von Interesse, wenn diese die hydrolytische Enzymaktivität nicht enthält, die erste Dicke e beizubehalten, und andererseits nach dem Aufbringen der wässrigen Lösung von Interesse, wenn diese die hydrolytische Enzymaktivität enthält, eine zweite Dicke e' aufzuweisen, die kleiner ist als die erste Dicke e,
   und dadurch, dass die erste Dicke e und/oder die zweite Dicke e' der transparenten Nachweisschicht (3) dafür ausgelegt sind, eine Farbe durch ein optisches Interferenzphänomen zu erzeugen, das durch Rekombination der an der ersten Grenzfläche (4) und an der zweiten Grenzfläche (5) reflektierten Lichtstrahlen hervorgerufen wird.

2. Kolorimetrische Vorrichtung nach Anspruch 1, **dadurch gekennzeichnet, dass** die erste Dicke e der Nachweisschicht (3) so ausgewählt ist, dass eine erste Farbe durch ein optisches Interferenzphänomen erzeugt wird, und dadurch, dass die Nachweisschicht (3) einerseits dafür ausgelegt ist, nach dem Aufbringen der wässrigen Lösung von Interesse, wenn diese die hydrolytische Enzymaktivität nicht enthält, die erste Dicke e, die die erste Farbe erzeugt, beizubehalten, und andererseits nach dem Aufbringen der wässrigen Lösung von Interesse, wenn diese die hydrolytische Enzymaktivität enthält, eine zweite Dicke e' aufzuweisen, die kleiner ist als die erste Dicke e, so dass entweder eine zweite Farbe durch ein optisches Interferenzphänomen erzeugt wird, die sich von der ersten Farbe unterscheidet, oder das Verschwinden der ersten Farbe hervorgerufen wird.

3. Kolorimetrische Vorrichtung nach einem der Ansprüche 1 oder 2, **dadurch gekennzeichnet, dass** die erste Dicke e der transparenten Nachweisschicht (3) eine gleichmäßige Dicke ist, die aus einem Dickenbereich zwischen 70 und 900 nm ausgewählt ist.

4. Kolorimetrische Vorrichtung nach einem der Ansprüche 1 bis 3, **dadurch gekennzeichnet, dass** die erste Dicke e und/oder die zweite Dicke e' der transparenten Nachweisschicht (3) dafür ausgelegt sind, eine Farbe durch ein optisches Interferenzphänomen mit einem maximalen Reflexionsvermögen bei einer Wellenlänge zwischen 380 nm und 780 nm zu erzeugen.

5. Kolorimetrische Vorrichtung nach einem der Ansprüche 1 bis 4, **dadurch gekennzeichnet, dass** die transparente Nachweisschicht (3) einen Brechungsindex $n_c$ zwischen 1,2 und 1,7 und vorzugsweise zwischen 1,4 und 1,6 besitzt.

6. Kolorimetrische Vorrichtung nach einem der Ansprüche 1 bis 5, **dadurch gekennzeichnet, dass** das in der transparenten Nachweisschicht (3) enthaltene Polymer aus Biopolymeren ausgewählt ist.

7. Kolorimetrische Vorrichtung nach einem der Ansprüche 1 bis 6, **dadurch gekennzeichnet, dass** das Substrat (2) transparent ist, und dadurch, dass die transparente Nachweisschicht (3) einen Brechungsindex n<sub>c</sub> besitzt, der sich von dem Brechungsindex n<sub>c</sub> des Substrats (2) unterscheidet.

8. Kolorimetrische Vorrichtung nach einem der Ansprüche 1 bis 6, **dadurch gekennzeichnet, dass** das Substrat (2) opak ist und eine reflektierende obere Oberfläche (2a) aufweist.

9. Kolorimetrische Vorrichtung nach einem der Ansprüche 1 bis 8, **dadurch gekennzeichnet, dass** das oder die in der transparenten Nachweisschicht (3) enthaltene(n) Polymere in immobilisierter Form vorliegt/vorliegen.

10. Kolorimetrische Vorrichtung nach Anspruch 9, **dadurch gekennzeichnet, dass** die transparente Nachweisschicht (3) Nanokristalle enthält, die Netzwerke von Wasserstoffbindungen mit dem Polymer von Interesse bilden.

11. Kolorimetrische Vorrichtung nach Anspruch 10, **dadurch gekennzeichnet, dass** die Nanokristalle aus Nanokristallen von Polysacchariden und vorzugsweise Cellulose-Nanokristallen bestehen.

12. Kolorimetrische Vorrichtung nach einem der Ansprüche 10 oder 11, **dadurch gekennzeichnet, dass** die Nanokristalle eine negative Oberflächenladung haben, dass die transparente Nachweisschicht (3) aus mindestens zwei Unterschichten (6) besteht, die jeweils das Polymer von Interesse enthalten, und dass die Polymer-Unterschichten (6) jeweils paarweise durch einen dazwischenliegende Unterschicht (7) getrennt sind, die eine Polykationverbindung enthält.

13. Kolorimetrische Vorrichtung nach einem der Ansprüche 10 oder 11, **dadurch gekennzeichnet, dass** die transparente Nachweisschicht (3) aus mindestens einem Paar von zwei Unterschichten (6, 7) besteht, die jeweils mindestens ein Polymer von Interesse enthalten, mit einer ersten Unterschicht (6), die ein erstes oder erste Polymere von Interesse umfasst, und einer zweiten Unterschichten (7), die ein zweites oder zweite Polymere von Interesse enthält.

14. Kolorimetrische Vorrichtung nach Anspruch 9, **dadurch gekennzeichnet, dass** die transparente Nachweisschicht (3) ein Harz enthält, das Netzwerke von kovalenten Bindungen mit dem Polymer von Interesse bildet.

15. Verfahren zum Nachweis, in einer wässrigen Lösung von Interesse, einer hydrolytischen Enzymaktivität gegenüber mindestens einem Polymer von Interesse, **dadurch gekennzeichnet, dass** es mindestens die Abfolge der folgenden Schritte umfasst:

   - Bereitstellung einer kolorimetrischen Vorrichtung (1) nach einem der Ansprüche 1 bis 14,
   - Aufbringen der wässrigen Lösung von Interesse auf die obere Oberfläche (3a) der transparenten Nachweisschicht (3),
   - Waschen der oberen Oberfläche (3a) der transparenten Nachweisschicht (3),
   - Trocknen der transparenten Nachweisschicht (3), und
   - Analyse der Farbe der transparenten Nachweisschicht (3) an der Stelle des Aufbringens der Lösung von Interesse.

**Claims**

1. Colorimetric device for detecting, in an aqueous solution of interest, a hydrolytic enzymatic activity with regard to at least one polymer of interest,
   which device (1) is **characterized in that** it comprises (i) a substrate (2) delimited at least by an upper surface (2a), and (ii) a transparent detection layer (3), having a first thickness e and including the said polymer of interest,
   which transparent detection layer (3) includes on the one hand, an upper surface (3a), to which it is intended to apply the said aqueous solution of interest and forming an initial interface (4) between the air and the detection layer (3), and on the other hand, a lower surface (3b), formed on the said upper surface (2a) of substrate (2) and together forming a second interface (5) between the detection layer (3) and the substrate (2),
   which upper surface (3a) of the transparent detection layer (3) is free, not covered in any way by an additional layer,
   which first interface (4) and which second interface (5) are apt to generate an optical reflection phenomenon,
   which detection layer (3) is adapted so that, on the one hand, after application of the said aqueous solution of interest, when it is deprived of the said hydrolytic enzymatic activity, it preserves the said first thickness e, and on the other hand, after the application of the said aqueous solution of interest, when it includes the said hydrolytic enzymatic activity, it has a second thickness e', thinner than the said first thickness e,

and wherein the said first thickness e and/or the said second thickness e' of the said detection layer (3) are adapted to generate a colour by an optical interference phenomenon caused by the recombining of the light beams reflected at the said first interface (4) and at the said second interface (5).

2. A colorimetric device according to claim 1, **characterized in that** the first thickness e of the detection layer (3) is chosen to generate a first colour by an optical interference phenomenon, **in that** the detection layer (3) is adapted so that, on the one hand, after application of the said aqueous solution of interest, when it is deprived of the said hydrolytic enzymatic activity, the said first thickness e generating the said first colour is preserved, and on the other hand, following the application of the said aqueous solution of interest when it includes the said hydrolytic enzymatic activity, it presents a second thickness e', thinner than first thickness e, producing either a second colour by an optical interference phenomenon, different from the said first colour, or causing the said first colour to disappear.

3. Colorimetric device according to any one of claims 1 or 2 **characterized in that** the first thickness e of the transparent detection layer (3) is a uniform thickness chosen from a range of thickness which is included between 70 and 900 nm.

4. Colorimetric device according to any one of claims 1 to 3 **characterized in that** the first thickness e and/or the second thickness e' of the said transparent detection layer (3) are adapted to generate a colour by an optical interference phenomenon with maximum reflectance for a wavelength included between 380 nm and 780 nm.

5. Colorimetric device according to any one of claims 1 to 4 **characterized in that** the transparent detection layer (3) has a refractive index $n_c$ included between 1.2 and 1.7, and preferably included between 1.4 and 1.6.

6. Colorimetric device according to any one of claims 1 to 5 **characterized in that** the polymer contained in the transparent detection layer (3) is chosen from among the biopolymers.

7. Colorimetric device according to any one of claims 1 to 6, **characterized in that** the substrate (2) is transparent and **in that** the transparent detection layer (3) has a refractive index $n_c$ which is different from the refractive index $n_s$ of the said substrate (2).

8. Colorimetric device according to any one of claims 1 to 6 **characterized in that** the substrate (2) is opaque and has a reflecting upper surface (2a).

9. Colorimetric device according to any one of claims 1 to 8 **characterized in that** the polymer or polymers contained in the transparent detection layer (3) is or are in an immobilized form.

10. Colorimetric device according to claim 9 **characterized in that** the transparent detection layer (3) contains nanocrystals forming hydrogen cross-linked networks with the polymer of interest.

11. Colorimetric device according to claim 10 **characterized in that** the nanocrystals consist of polysaccharide nanocrystals and preferably of cellulose nanocrystals.

12. Colorimetric device according to any one of claims 10 or 11, **characterized in that** the nanocrystals have a negative charge, **in that** the transparent detection layer (3) consists of at least two sub-layers (6) each containing the polymer of interest, and **in that** the said polymer sub-layers (6) are separated in pairs by an interposed sub-layer (7) containing a poly-cationic compound.

13. Colorimetric device according to any one of claims 10 or 11, **characterized in that** the transparent detection layer (3) consists of at least one pair of two sub-layers (6, 7) each containing at least one polymer of interest, with a first sub-layer (6) having one first polymer or first polymers of interest, and one second sub-layers (7) containing a second polymer or second polymers of interest.

14. Colorimetric device according to claim 9 **characterized in that** the transparent detection layer (3) contains a resin forming covalent cross-linked networks with the polymer of interest.

15. A process for detecting, in an aqueous solution of interest, hydrolytic enzymatic activity with regard to at least one polymer of interest, **characterised in that** it comprises at least the following succession of steps:

- the supply of a colorimetric device (1) according to any one of claims 1 to 14,

- the application of the said aqueous solution of interest to the upper surface (3a) of the said transparent detection layer (3),
- the washing of the said upper surface (3a) of the transparent detection layer (3),
- the drying of the said transparent detection layer (3), and
- the analysis of the colour of the said transparent detection layer (3) at the application site of said solution of interest.

Figure 1

Figure 2

6a    3a, 4    7a    7b    1

6b    3

e    2

2a, 3b, 5

Figure 3

6b    3a, 4    7a    7b    1

e'    3

2a, 3b, 5    2

Figure 4

Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2004031760 A

- US 2010068749 A

**Littérature non-brevet citée dans la description**

- **NELSON et al.** *J. Biol. Chem.,* 1944, vol. 153, 375-380
- **DUCÉRÉ et al.** A capacitive humidity sensor using cross-linked cellulose acetate butyrate. *Sensors and Actuators B: Chemical,* 2005, vol. 106 (1), 331-334
- **WU et al.** Molecularly imprinted organic-inorganic hybrid membranes for selective séparation of phenylalanine isomers and its analogue. *Séparation and Purification Technology,* 2009, vol. 68 (1), 97-104
- **SCHULER et al.** Decomposable hollow biopolymer-based capsules. *Biomacromolecules,* 2001, vol. 2 (3), 921-926
- **REVOL et al.** *Int. J. Biol. Macromol.,* 1992, vol. 14, 170-172
- **CRANSTON et al.** *Biomacromolecules,* 2006, vol. 7, 2522

- **JEAN et al.** Structural Details of Cellulose Nanocrystals/Polyelectrolytes Multilayers Probed by Neutron Reflectivity and AFM. *Langmuir,* 2008, vol. 24 (7), 3452-3458
- **CRANSTON et al.** Morphological and Optical Characterization of Polyelectrolyte Multilayers Incorporating Nanocrystalline Cellulose. *Biomacromolecules,* 2006, vol. 7 (9), 2522-2530
- **WAGBERG et al.** The Build-Up of Polyelectrolyte Multilayers of Microfibrillated Cellulose and Cationic Polyelectrolytes. *Langmuir,* 2008, vol. 24 (3), 784-795
- **CRANSTON et al.** Birefringence in Spin-Coated Films Containing Cellulose Nanocrystals. *Colloids and Surfaces A,* 2008, vol. 325 (1-2), 44-51
- **DAVID.** Spin Coating of Thin and Ultrathin Polymer Films. *Polymer engineering and science,* Décembre 1998, vol. 38 (12), 2040